(19) **Europäisches Patentamt**
**European Patent Office**
**Office européen des brevets**

(11) **EP 1 453 508 B1**

(12) **EUROPEAN PATENT SPECIFICATION**

(45) Date of publication and mention
of the grant of the patent:
**28.03.2012 Bulletin 2012/13**

(51) Int Cl.:
*A61K 31/445* (2006.01)    *A61P 35/00* (2006.01)

(21) Application number: **02786964.3**

(22) Date of filing: **06.12.2002**

(86) International application number:
**PCT/US2002/039261**

(87) International publication number:
**WO 2003/053442 (03.07.2003 Gazette 2003/27)**

(54) **TEMPAMINE COMPOSITIONS AND USE AGAINST CELLULAR OXIDATIVE DAMAGE**

ZUSAMMENSETZUNGEN ENTHALTEND TEMPAMINE UND DEREN VERWENDUNG ZUM
SCHUTZ VOR OXIDATIVER SCHÄDIGUNG

COMPOSITIONS DE TEMPAMINE ET LEUR UTILISATION POUR LA PROTECTION CONTRE LE
STRESS OXYDATIF CELLULAIRE

(84) Designated Contracting States:
**AT BE BG CH CY CZ DE DK EE ES FI FR GB GR
IE IT LI LU MC NL PT SE SI SK TR**

(30) Priority: **06.12.2001 US 338046 P**

(43) Date of publication of application:
**08.09.2004 Bulletin 2004/37**

(73) Proprietor: **YISSUM RESEARCH DEVELOPMENT
COMPANY
OF THE HEBREW UNIVERSITY OF JERUSALEM
91390 Jerusalem (IL)**

(72) Inventors:
• **BARENHOLZ, Yechezkel**
**93707 Jerusalem (IL)**
• **WASSERMAN, Veronica**
**Thornhill, Ontario L4J 5W5 (CA)**

(74) Representative: **Vossius & Partner
Siebertstrasse 4
81675 München (DE)**

(56) References cited:
**WO-A-02/26231      WO-A-97/41826
US-A- 5 827 532**

• **SAMUNI, A. ET AL.: "Nitroxide stable radiclas
protect beating cardiomyocytes against
oxidative damage" JOURNAL OF CLINICAL
INVESTIGATION, vol. 87, no. 5, - May 1991
(1991-05) pages 1526-1530, XP009006522**
• **WANG, G. ET AL.: "Opposing effects of nitroxide
free radicals in Escherichia coli mutants deficient
in DNA repair" BIOCHIMICA ET BIOPHYSICA
ACTA, vol. 1305, - 1996 pages 71-78,
XP009006539**
• **SAMUNI, A. ET AL.: "Nitroxides block DNA
scission and protect cells from oxidative
damage" BIOCHEMISTRY, vol. 30, - 1991 pages
555-561, XP001146052**
• **KRISHNA, M. C. ET AL.: "Studies of structure-
activity relationship of nitroxide free radicals and
their precursors as modifiers against oxidative
damage" J.MED.CHEM., vol. 41, - 1998 pages
3477-92, XP001146049**
• **SAMUNI, A. ET AL.: "Superoxide reaction with
nitroxides" FREE RADICAL RESEARCH
COMMUNICATIONS, vol. 9, no. 3-6, - 1990 pages
241-9, XP009006540**
• **RACHMILEWITZ, D. ET AL.: "Sulphydryl blocker
induced small intestinal inflammation in rats: a
new model mimicking Crohn's disease" GUT, vol.
41, no. 3, - 1997 pages 358-365, XP009006553**
• **DAMIANI, E. ET AL.: "The effects of nitroxide
radicals on oxidative DNA damage" FREE
RADICAL BIOLOGY & MEDICINE, vol. 28, no. 8, -
2000 pages 1257-65, XP001146053**

## Description

### Field of the Invention

The present invention relates to the therapeutic use of tempamine for treating conditions caused by cellular oxidative damage or cellular oxidation stress. In a particular embodiment, the invention relates to a liposome composition having entrapped tempamine.

### Background of the Invention

Species capable of independent existence that contain one or more unpaired electrons are commonly referred to as free radicals. There are many types of free radicals, differing in their reactivity, origin, place of formation, degree of lipophilicity, and potential biological target. In recent years, the term "reactive oxygen species" (ROS) has been adopted to include molecules such as hypochlorous acid (HOCl), singlet oxygen ($^1O_2$), and hydrogen peroxide ($H_2O_2$), which are not radicals in nature but are capable of radical formation in the extra- and intracellular environments (Halliwell, B. and Gutteridge, J.M.C (Eds), FREE RADICAL IN BIOLOGY AND MEDICINE, 2nd Ed. Clarendon Press, Oxford, 1989).

ROS are involved in many biological processes, including regulating biochemical processes, assisting in the action of specific enzymes, and removing and destroying bacteria and damaged cells (Halliwell, B. and Gutteridge, J.M.C (Eds), FREE RADICAL IN BIOLOGY AND MEDICINE, 2nd Ed. Clarendon Press, Oxford, 1989). Free radicals are essential for the body and under normal circumstances there is a balance between oxidative and reductive compounds (redox state) inside the cell. If the balance is impaired in favor of oxidative compounds, oxidative stress is said to occur (Parke, et al., Int. J. Occup. Med. Environ. Health 9:331-340 (1996); Knight, Ann. Clin. Lab. Sci. 27:11-25 (1997); Stohs, J. Basic. Clin. Physiol. Pharmacol. 6: 205-228 (1995)). Oxidative stress may occur as a result of oxidative insults such as air pollution or the "oxidative burst" characteristic of activated neutrophils mediated by the immune response. A constant source of oxidative stress results from formation of superoxide anion via "electron leakage" in the mitochondria during production of adenosine triphosphate (ATP). Although superoxide anion is not exceedingly reactive in and of itself, it can initiate a chain of events that eventually results in the formation of the highly reactive free radicals and other oxidants. If these reactive oxygen species are not controlled by enzymatic and/or non-enzymatic antioxidant systems, *in vivo* oxidation of critical cellular components such as membranes, DNA, and proteins will result, eventually leading to tissue damage and dysfunction.

Reactive oxygen species (ROS) have been implicated in the development of many disorders. ROS are involved in artherosclerotic lesions, in the evolution of various neurodegenerative diseases, and are also produced in association to epileptic episodes, in inflammation, in the mechanisms of action of various neurotoxicants, or as side effects of drugs.

It is clear that the balance between oxidative and reductive compounds in biological systems is important. To preserve this balance the body has a number of protective antioxidant mechanisms that remove or prevent formation of ROS. There are also mechanisms that repair damage caused by ROS *in vivo.* Defense systems include enzymatic as well as non-enzymatic antioxidant components. However, the development of methods and compounds to combat oxidative stress or toxicity associated with oxygen-related species has enjoyed limited success.

Two main pathological conditions connected to oxidative stress are cell damage and malignancy. The role of reactive oxygen species (ROS) in degradative cell damage has been studied (Samuni, A. et al., Free Radical Res. Commun. 12-13:187-194 (1991); Samuni, A. et al, J. Clin. Invest 87:1526-1530 (1991); Burton, G.W. et al., J. Am. Chem. Soc. 103:6478-6485 (1981)), however, their role in tumor proliferation still remains unclear (Mitchell, J.B., et al., Arch. Biochem. Biophys. 298:62-70 (1991)). It is accepted that apoptosis and cancer are opposing phenomena, but ROS have been shown to play a key role in both (Mates, J.M. et al., Int. J. Biochem. Cell Biol. 32:157-170 (2000)).

Many types of cancer cells have an altered oxidant level (Wiseman, H. et al., Biochem. J. 313:17-29 (1996)) and several tumors that have been strongly associated with the oxidant-antioxidant imbalance, including bladder, blood, bowel, breast, colorectal, liver, lung, kidney, esophagus, ovary, prostate, and skin. The generation of large amounts of reactive oxygen intermediates in cancer cells may contribute to the ability of some tumors to mutate, inhibit antiproteases, and injure local tissues, thereby promoting tumor heterogeneity, invasion, and metastasis (Mates, J.M. et al., Int J. Biochem. Cell Biol. 32:157-170 (2000); Szatrowski, T.P. et al., Cancer Res. 51:794-798 (1991)). The pro-oxidant state also provides tumor cells with a survival advantage over normal cells during chemotherapy. For example, the presence of high $H_2O_2$ concentration inhibits the ability of different anti-cancer drugs (etopside, doxorubicin, cisplatin, taxol, and AraC) to induce apoptosis (Shacter, E., et al., Blood. 96:307-313 (2000)). Similarly, relatively low concentrations of $H_2O_2$ (50-100 $\mu$M) inhibit the induction of apoptosis by the chemotherapy drug etopsid and calcium ionophore A23187 (Lee, Y-J. et al., J. Biol. Chem. 274:19792-19798 (1999)). The presence of $H_2O_2$ not only reduces the overall cytotoxicity of tested drugs but also shifts type of cell death from apoptosis to necrosis. The shift from apoptotic death to necrosis is important, since cells which undergo apoptosis are capable of being recognized and phagocytosed by monocyte-derived macrophages before losing the membrane permeability barrier (*Id.*). In contrast, necrotic cells are not phagocytosed until they have begun to leak their contents into the extracellular space, thus inducing an inflammatory response, which may interfere with chemotherapy (Savill, J., et al., Immunol. Today, 14:131-136 (1993)).

The use of antioxidants, such as α-tocopherol, desferal, and nitroxides, in cancer therapy has been explored (Chenery, R., et al., Nat. Med. 3:1233-1241 (1997); Shacter, E., et al., Blood. 96:307-313 ((2000)). However, the fast clearance of antioxidants when administered in free form and their chemical degradation in plasma limit their effectiveness *in vivo*.

There are a variety of approaches to extending the blood circulation time of therapeutic agents, such as modifying the drug with polymer chains (U.S. Patent No. 4,179,337). Another approach is to entrap the agent in a liposome. For effective therapy, it is desirable to load a high concentration of the therapeutic agent in the liposome. Also, the rate of leakage of the agent from the liposomes should be low. There are a variety of drug-loading methods available for preparing liposomes with entrapped drug, including passive entrapment and active remote loading. The passive entrapment method is most suited for entrapping a high concentration of lipophilic drugs in the liposome and for entrapping drugs having a high water solubility.

In the case of ionizable hydrophilic or amphipathic drugs, even greater drug-loading efficiency can be achieved by loading the drug into liposomes against a transmembrane ion gradient (Nichols, J.W., et al., Biochim. Biophys. Acta 455:269-271 (1976); Cramer, J., et al., Biochemical and Biophysical Research Communications 75(2):295-301 (1977)). This loading method, generally referred to as remote loading, typically involves a drug having an ionizable amine group which is loaded by adding it to a suspension of liposomes prepared to have a lower inside/higher outside ion gradient, often a pH gradient.

However, there are recognized problems with remote loading, one being that not all ionizable drugs accumulate in the liposomes in response to an ion gradient (Chakrabarti, A., et al., U.S. Patent No. 5,380,532; Madden, T.D., et al., Chemistry and Physics of Lipids 53:37-46 (1990)). Another problem is that some agents which do accumulate in the liposomes are immediately released after accumulation. Yet another problem is that some agents which are successfully loaded and retained in the liposome *in vitro* have a high leakage rate from the liposomes *in vivo,* obviating the advantages of administering the agent in liposome-entrapped form.

## Summary of the Invention

Accordingly, it is an object of the invention to provide a composition effective to treat conditions caused by cellular oxidative damage.

It is another object of the invention to provide a tempamine composition having a blood circulation lifetime sufficiently long to achieve a therapeutic effect to treat conditions caused by cellular oxidative damage.

It is a further object of the invention to provide a use of tempamine for treating a condition resulting from oxidative stress or damage.

It is still another object of the invention to provide a composition comprised of tempamine in liposome-entrapped form.

These and other objects and features of the invention will be more fully appreciated when the following detailed description of the invention is read in conjunction with the accompanying drawings.

## Brief Description of the Drawings

Figs. 1A-1C show the chemical structures of the piperidine nitroxides tempo (Fig. 1A), tempol (Fig. 1 B), and tempamine (Fig. 1 C);

Fig. 2 shows the redox states of the nitroxide aminoxyl moiety;

Fig. 3 shows the typical electron paramagnetic resonance (EPR) signal of tempamine;

Fig. 4 is a plot of percent survival of MCF-7 human breast carcinoma cells after 72 hours of exposure to various concentrations of tempamine (closed circles) or tempol (open circles);

Figs. 5A-5B are flow cytometry scans of MCF-7 cancer cells in a buffer (control, Fig. 5A) and treated with tempamine (1 mM) for 24 hours (Fig. 5B), trypsinized, stained with merocyanine-540 and then analyzed by flow cytometry. The area designated by M1 indicates fluorecently-labeled apoptotic cells;

Fig. 6 illustrates the ionization events in loading the exemplary nitroxide tempamine (TMN) into liposomes against an ammonium ion gradient;

Fig. 7 shows the electron paramagnetic resonance (EPR) signal of tempamine before (dashed line) and after (solid line) encapsulation into liposomes;

Fig. 8 shows the cyclic voltammetry (CV) signal of tempamine before (dashed line) and after (solid line) encapsulation into liposomes;

Figs. 9A-9B are plots showing the leakage of tempamine from liposomes prepared from egg phosphatidylcholine (Fig. 9A) and from hydrogenated soy phosphatidylcholine (Fig. 9B) at 4°C (squares), 25°C (open circles) and 37°C (closed circles);

Fig. 10 is a plot showing the percent encapsulation and stability of four tempamine-loaded liposomal formulations as a function of lipid composition and liposome size. The percent encapsulation of tempamine. immediately after liposome preparation (dotted bars), after 2 months storage in saline at 4°C (hatched bars), after 15 hours storage in saline (hotizontal stripes), and after 15 hours in plasma at 37°C (white bars) is shown;

Fig. 11 is a plot showing the plasma elimination of free tempamine (closed circles) and liposome-entrapped tempamine (open circles) as a function of time after intravenous administration of 18 mg (100 µmole)/kg of free tempamine or liposome-entrapped tempamine;

Figs. 12A-12F are plots showing the distribution of liposome-entrapped tempamine (open circles) and of the liposomal lipid label (closed circles) in mice injected intravenously with liposome-entrapped tempamine as a function of time post injection in mouse plasma (Fig. 12A), liver (Fig. 12B), spleen (Fig. 12C), kidney (Fig. 12D), lung (Fig. 12E), and tumor (Fig. 12F);

Figs. 13A-13F are plots showing the tempamine to phospholipid ratio in plasma (Fig. 13A), liver (Fig. 13B), spleen (Fig. 13C), kidney (Fig. 13D), lung (Fig. 13E), and tumor (Fig. 13F) at various times post injection;

Fig. 14 is a plot showing the amount of liposome phospholipid per gram tissue following administration of liposomes containing entrapped tempamine to healthy rats (closed circles) and to rats having induced adjuvent arthritis (open circles); and

Figs. 15A-15B are bar graphs showing the tissue distribution of liposome-entrapped tempamine, taken as nmole phospholipid (PL)/gram tissue, in healthy rats (Fig. 15A) and in rats having induced adjuvent arthritis (Fig. 15B) at 4 hours (dotted bars), 24 hours (hatched bars), 48 hours (horizontal stripes) and 72 hours (white bars).

**Detailed Description of the Invention**

I. Definitions

The term "nitroxide" is used herein to describe stable nitroxide free radicals, their precursors, and their derivatives thereof including the corresponding hydroxylamine derivative where the oxygen atoms are replaced with a hydroxyl group and exist in hydrogen halide form. In the nitroxides described here, the unpaired electron of a nitroxide is stable in part because the nitrogen nucleus is attached to two carbon atoms which are substituted with strong electron donors. With the partial negative charge on the oxygen of the N-0 bond, the two adjacent carbon atoms together localize the unpaired electron on the nitrogen nucleus. Nitroxides may have either a heterocyclic or linear structure. The fundamental criterion is a stable free radical.

The term "nitroxide having a protonable amine" intends a nitroxide having a primary, secondary or tertiary amine capable of accepting at least one hydrogen proton.

"TMN" as used herein refers to tempamine.

II. Nitroxides

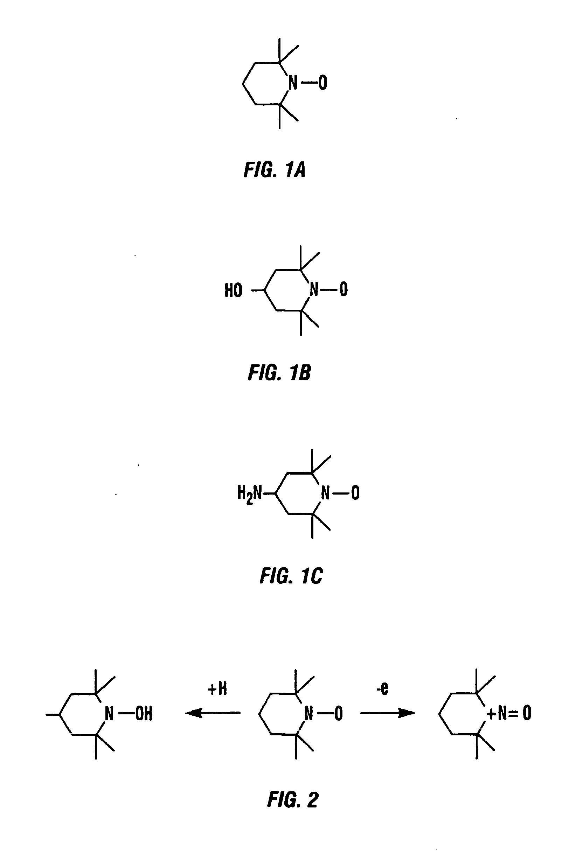

Piperidine nitroxides are chemically stable, *n,n*-disubstituted >NO radicals. Figs. 1A-1C show the chemical structures of tempo (Fig. 1A), tempol (Fig. 1B), and tempamine (Fig. 1 C). These cyclic radicals are cell permeable, nontoxic, and nonimmunogenic (Afzal, V., et al., Invest. Raiol. 19:549-552 (1984); Ankel, E.G., et al., Life Sci. 40:495-498 (1987); DeGraff, W.G., et al., Environ. Mol. Mutagen. 19:21-26 (1992)). Among antioxidants, nitroxides are unusual in their mode of action being mainly oxidants and not reductants (Mitchell, J.B., et al., Arch. Biochem. Biophys. 298:62-70 (1991); Samuni, A.et al., Free Radical. Res. Commun. 12-13:187-194 (1991)). They also possess the ability to be at least partly regenerated (*Id.*). Nitroxides exert their antioxidant activity through several mechanisms: SOD-mimic, oxidation of reduced metal ions, reduction of hypervalent metals and interruption of radical chain reactions (Mitchell, J.B., et al., Tempol. Arch. Biochem. Biophys. 298: 62-70 (1991); Samuni, A. et al., Free Radical. Res. Commun. 12-13:187-194 (1991); Krishna, M.C., et al., Proc Natl Acad Sci U S A. 89:5537-5541 (1992)).

Nitroxides are widely utilized as spin labels due to their amphipathy and chemical stability as radicals (Kocherginsky, N. and Swartz, H.M., NITROXIDE SPIN LABELS: REACTIONS IN BIOLOGY AND CHEMISTRY, Boca Raton, FL: CRC Press (1995)). The paramagnetism of the nitroxide is lost when it is oxidized or reduced, and its EPR signal disappears. Nitroxides may be reduced to hydroxylamines and may be oxidized to oxo-ammonium cations, as shown in Fig. 2. This fast reduction *in vivio* to hydroxylamines and their rapid clearance from blood limits their effectiveness as therapeutic agents.

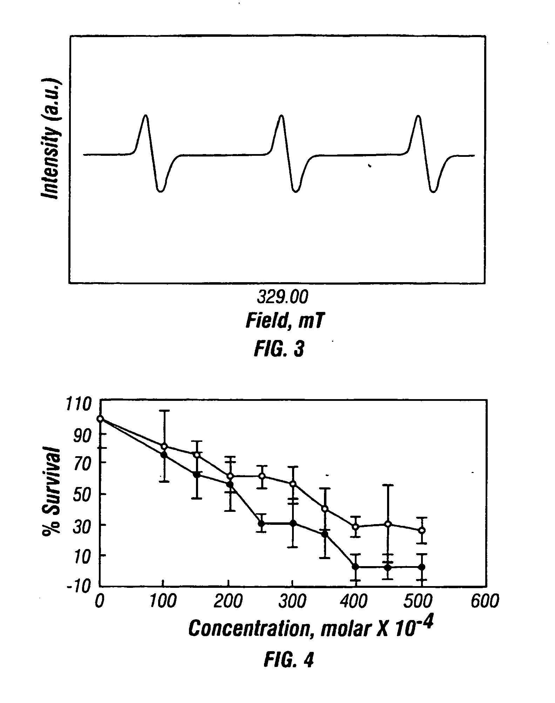

The electron paramagnetic resonance (EPR) signal of free tempamine is shown in Fig. 3. Free tempamine gives well-defined peaks in both solutions of n-octanol and water. The peak area (or height) is proportional to the tempamine concentrations in the aqueous phase and in n-octanol. The tempamine concentration in both solvents was determined from a calibration curve of tempamine in each of the two solvents n-octanol and water.

A. *In vitro* Cytotoxicity of Tempamine

In studies performed in support of the present invention, tempamine was tested *in vitro* to determine if it exhibits anti-proliferative or cellular anti-growth activity. As described in Example 1, the cytotoxicity of tempamine was determined on three cell lines, MCF-7 (human breast adenocarcinoma), M-109S (doxorubicin-sensitive human breast carcinoma), and M-109R (doxorubicin-resistant human breast carcinoma). The effect of free tempamine on cell proliferation was determined by a methylene blue assay as described in Example 1 B.

Fig. 4 is a plot comparing the percent survival of MCF-7 human breast carcinoma cells at various doses of tempamine (closed circles) and tempol (open circles) *in vitro*. The cells were analyzed after 72 hours of exposure to the nitroxides. Tempamine and tempol cell growth inhibitory activities were of similar magnitude. The $IC_{50}$ of tempamine was 210 $\mu$M and the $IC_{50}$ of tempol was 320 $\mu$M.

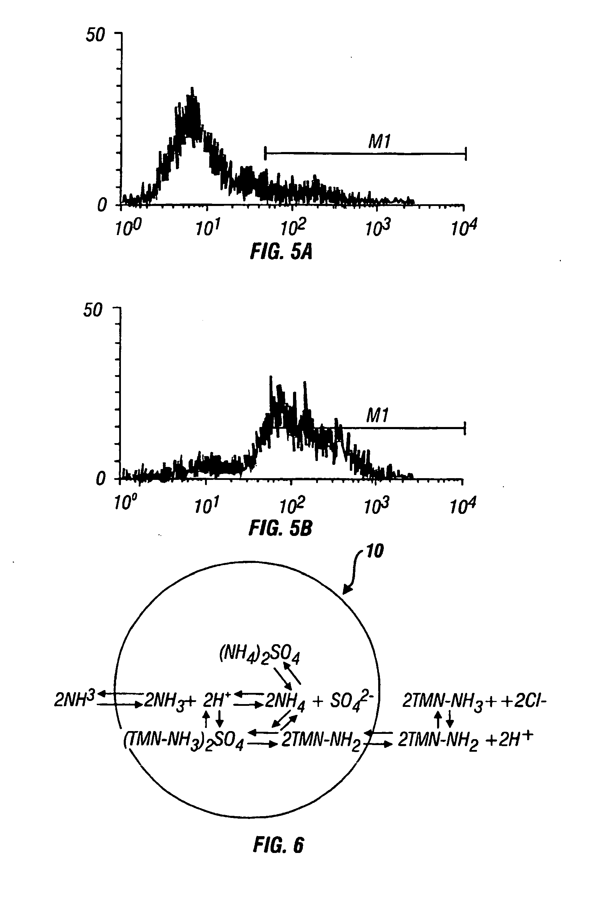

In another study, the MCF-7 cell line was used to investigate the mechanism of growth inhibition by tempamine. Untreated cells and tempamine-treated cells (24-hours exposure) were contacted with merocyanine-540. Merocyanine-540 binds selectively to phophatidylserine, which appears at the external surface of the cell at the beginning of apoptosis and is therefore one of the apoptotic markers (Reid, S., et al., J. Immunol. Methods 192:43 (1996)). After interaction with merocyanine-540, cells were analyzed by flow cytometry as described in the methods section below. The results are shown in Figs. 5A-5B where a FACscan of the untreated control cells is shown in Fig. 5A and a FACscan of cells treated with 1 mM tempamine for 24 hours is shown in Fig. 5B. After the 24 hour treatment period, the cells were trypsinized, stained with merocyanine-540 and analyzed by flow cytometry. The area designated by M1 in Figs. 5A-5B indicates fluorescently-labeled apoptotic cells. The scans show that most of the cells (77%) after tempamine treatment were fluorescently labeled, compared to 14% fluorescently labeled without tempamine treatment. This result indicates that tempamine kills cancer cells via apoptosis induction.

Tempamine cytotoxicity on two additional cell lines, M-109S and M-109R, had similar cytoxicity values, with an $IC_{50}$ of tempamine around 700 $\mu$M, significantly greater than the $IC_{50}$ of the MCF-7 cells (210 $\mu$M).

The cytotoxicity data shows that tempamine in free form has therapeutic activity. Tempamine was effective to inhibit the cell growth of breast carcinoma cells. The growth inhibition was achieved by apoptosis, which as discussed in the background section, is desirable since cells which undergo apoptosis are capable of being recognized and phagocytosed by monocyte-derived macrophages before losing the membrane permeability barrier. In contrast, cells with die by necrosis are not phagocytosed until they have begun to leak their contents into the extracellular space, thus inducing an inflammatory response, which may interfere with chemotherapy.

III. Tempamine Liposome Composition

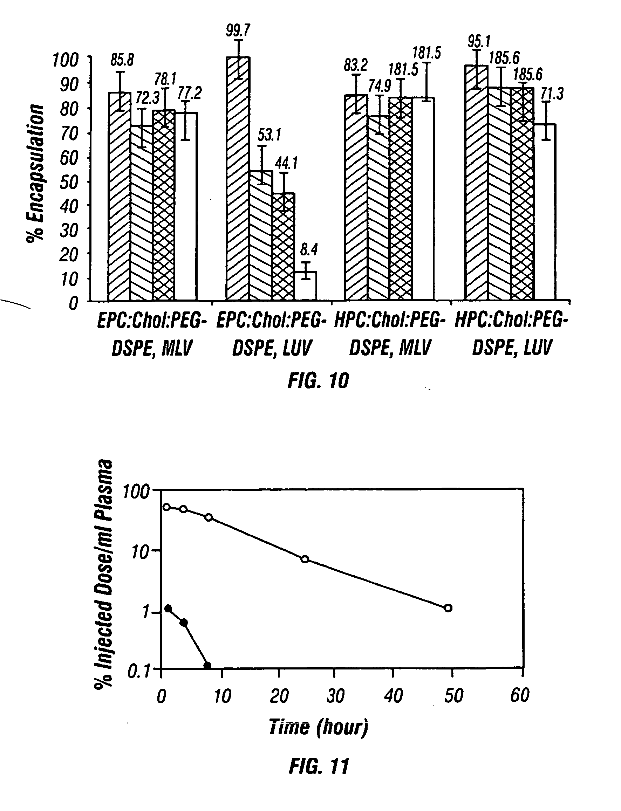

Accordingly, the invention includes, in one aspect, a composition effective to treat a condition caused by oxidative damage. The composition includes tempamine in a pharmaceutically-acceptable medium in an amount effective to reverse or ameleoriate the symptoms associated with cellular oxidative damage or stress. As will be described below with respect to Fig. 11, tempamine in free form, like other nitroxides, have a short blood circulation lifetime ($_{1/2}$). It is desirable, therefore, to provide a tempamine composition where tempamine is formulated to extend its blood circulation lifetime. There are a variety of formulations suitable, such as providing a coating of polymer chains or lipid chains around the compound. In a preferred embodiment of the invention, tempamine is entrapped in liposomes. In studies now to be described, liposome-entrapped tempamine was characterized to determine the percent of encapsulation of tempamine, the *in vitro* release rate of tempamine and the *in vitro* plasma stability. In still other studies the *in vivo* plasma clearance, tissue distribution and release rate of the liposomes were determined.

A. Liposome Composition

Liposomes suitable for use in the composition of the present invention include those composed primarily of vesicle-forming lipids. Vesicle-forming lipids, exemplified by the phospholipids, form spontaneously into bilayer vesicles in water. The liposomes can also include other lipids incorporated into the lipid bilayers, with the hydrophobic moiety in contact with the interior, hydrophobic region of the bilayer membrane, and the head group moiety oriented toward the exterior, polar surface of the bilayer membrane.

The vesicle-forming lipids are preferably ones having two hydrocarbon chains, typically acyl chains, and a head group, either polar or nonpolar. There are a variety of synthetic vesicle-forming lipids and naturally-occurring vesicle-forming lipids, including the phospholipids, such as phosphatidylcholine, phosphatidylethanolamine, phosphatidic acid, phosphatidylinositol, and sphingomyelin, where the two hydrocarbon chains are typically between about 14-24 carbon atoms in length, and have varying degrees of unsaturation. The above-described lipids and phospholipids whose acyl chains have varying degrees of saturation can be obtained commercially or prepared according to published methods. Other suitable lipids include glycolipids and sterols such as cholesterol.

Cationic lipids (mono and polycationic) are also suitable for use in the liposomes of the invention, where the cationic lipid can be included as a minor component of the lipid composition or as a major or sole component. Such cationic lipids typically have a lipophilic moiety, such as a sterol, an acyl or diacyl chain, and where the lipid has an overall net positive charge. Preferably, the head group of the lipid carries the positive charge. Exemplary of mono cationic lipids include 1,2-dioleyloxy-3-(trimethylamino) propane (DOTAP); N-[1-(2,3-ditetradecyloxy)propyl]-N,N-dimethyl-N-hydroxyethylammonium bromide (DMRIE); N-[1-(2,3,-dioleyloxy)propyl]-N,N-dimethyl-N-hydroxy ethylammonium bromide (DORIE); N-[1-(2,3-dioleyloxy) propyl]-N,N,N-trimethylammonium chloride (DOTMA); 3β[N-(N',N'-dimethylaminoethane) carbamoly] cholesterol (DC-Chol); and dimethyldioctadecylammonium (DDAB).

Examples of polycationic lipids include lipids having a similar lipopholic group as described above for the monocationic lipids and a polycationic moiety attached thereto. Exemplary polycationic moieties include spermine or spermidine (as exemplified by DOSPA and DOSPER), or a peptide, such as polylysine or other polyamine lipids. For example, the neutral lipid (DOPE) can be derivatized with polylysine to form a cationic lipid.

The cationic vesicle- may also include a neutral lipid, such as dioleoylphosphatidyl ethanolamine (DOPE) or cholesterol. These lipids are sometimes referred to as helper lipids.

The vesicle-forming lipid can be selected to achieve a specified degree of fluidity or rigidity, to control the stability of the liposome in serum and to control the rate of release of the entrapped agent in the liposome. Liposomes having a more rigid lipid bilayer, in the gel (solid ordered) phase or in a liquid crystalline (liquid disordered) bilayer, are achieved by incorporation of a relatively rigid lipid, e.g., a lipid having a relatively high phase transition temperature, e.g., above room temperature, more preferably above body temperature and up to 80°C. Rigid, i.e., saturated, lipids contribute to greater membrane rigidity in the lipid bilayer. Other lipid components, such as cholesterol, are also known to contribute to membrane rigidity in lipid bilayer structures and can reduce membrane free volume thereby reducing membrane permeability.

Lipid fluidity is achieved by incorporation of a relatively fluid lipid, typically one having a lipid phase with a relatively low liquid to liquid-crystalline phase transition temperature, e.g., at or below room temperature, more preferably, at or below body temperature.

The liposomes also include a vesicle-forming lipid derivatized with a hydrophilic polymer. As has been described, for example in U.S. Patent No. 5,013,556 and in WO 98/07409, such a hydrophilic polymer provides a surface coating of hydrophilic polymer chains on both the inner and outer surfaces of the liposome lipid bilayer membranes. The outermost surface coating of hydrophilic polymer chains is effective to provide a liposome with a long blood circulation lifetime *in vivo*. The inner coating of hydrophilic polymer chains extends into the aqueous compartments in the liposomes, *i.e.*, between the lipid bilayers and into the central core compartment, and is in contact with any entrapped agents. Vesicle-forming lipids suitable for derivatization with a hydrophilic polymer include any of those lipids listed above, and, in particular phospholipids, such as distearoyl phosphatidylethanolamine (DSPE).

Hydrophilic polymers suitable for derivatization with a vesicle-forming lipid include polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxyethylacrylate, hydroxymethylcellulose, hydroxyethylcellulose, polyethyleneglycol, and polyaspartamide. The polymers may be employed as homopolymers or as block or random copolymers.

A preferred hydrophilic polymer chain is polyethyleneglycol (PEG), preferably as a PEG chain having a molecular weight between about 500 and about 12,000 Daltons, (g/mol) more preferably between about 500 and about 5,000 Daltons, most preferably between about 1,000 to about 5,000 Daltons. Methoxy or ethoxy-capped analogues of PEG are also preferred hydrophilic polymers, commercially available in a variety of polymer sizes, e.g., 120-20,000 Daltons.

Preparation of vesicle-forming lipids derivatized with hydrophilic polymers has been described, for example in U.S. Patent No. 5,395,619. Preparation of liposomes including such derivatized lipids has also been described, where typically, between 1-20 mole percent of such a derivatized lipid is included in the liposome formulation. It will be appreciated that the hydrophilic polymer may be stably coupled to the lipid, or coupled through an unstable linkage which allows the coated liposomes to shed the coating of polymer chains as they circulate in the bloodstream or in response to a stimulus, as has been described, for example, in U.S. Patent No. 6,043,094.

B. Liposome Preparation

In the present invention, a preferred method of preparing the liposomes is by remote loading. In the studies performed in support of the invention, the exemplary nitroxide tempamine was loaded into pre-formed liposomes by remote loading against an ion concentration gradient, as has been described in the art (U.S. Patent No. 5,192,549) and as described in Example 2. In a remote loading procedure, a drug is accumulated in the intraliposome aqueous compartment at concentration levels much greater than can be achieved with other loading methods.

Liposomes having an ion gradient across the liposome bilayer for use in remote loading can be prepared by a variety of techniques. A typical procedure is as described above, where a mixture of liposome-forming lipids is dissolved in a suitable organic solvent and evaporated in a vessel to form a thin film. The film is then covered with an aqueous medium containing the solute species that will form the aqueous phase in the liposome interior spaces.

After liposome formation, the vesicles may be sized to achieve a size distribution of liposomes within a selected range, according to known methods. The liposomes are preferably uniformly sized to a selected size range between 0.04 to 0.25 μm. Small unilamellar vesicles (SUVs), typically in the 0.04 to 0.08 μm range, can be prepared by extensive sonication or homogenization of the liposomes. Homogeneously sized liposomes having sizes in a selected range between about 0.08 to 0.4 microns can be produced, e.g., by extrusion through polycarbonate membranes or other defined pore size membranes having selected uniform pore sizes ranging from 0.03 to 0.5 microns, typically, 0.05, 0.08, 0.1, or 0.2 microns. The pore size of the membrane corresponds roughly to the largest size of liposomes produced by extrusion through that membrane, particularly where the preparation is extruded two or more times through the same membrane. The sizing is preferably carried out in the original lipid-hydrating buffer, so that the liposome interior spaces retain this medium throughout the initial liposome processing steps.

After sizing, the external medium of the liposomes is treated to produce an ion gradient across the liposome membrane, which is typically a lower inside/higher outside ion concentration gradient. This may be done in a variety of ways, e.g., by (i) diluting the external medium, (ii) dialysis against the desired final medium, (iii) molecular-sieve chromatography, e.g., using Sephadex G-50, against the desired medium, or (iv) high-speed centrifugation and resuspension of pelleted liposomes in the desired final medium. The external medium which is selected will depend on the mechanism of gradient formation and the external pH desired, as will now be considered.

In the simplest approach for generating an ion gradient, the hydrated, sized liposomes have a selected internal-medium pH. The suspension of the liposomes is titrated until a desired final pH is reached, or treated as above to exchange the external phase buffer with one having the desired external pH. For example, the original medium may have a pH of 5.5, in a selected buffer, e.g., glutamate or phosphate buffer, and the final external medium may have a pH of 8.5 in the same or different buffer. The internal and external media are preferably selected to contain about the same osmolarity, e.g., by suitable adjustment of the concentration of buffer, salt, or low molecular weight solute, such as sucrose.

In another general approach, the gradient is produced by including in the liposomes, a selected ionophore. To illustrate, liposomes prepared to contain valinomycin in the liposome bilayer are prepared in a potassium buffer, sized, then exchanged with a sodium buffer, creating a potassium inside/sodium outside gradient. Movement of potassium ions in an inside-to-outside direction in turn generates a lower inside/higher outside pH gradient, presumably due to movement of protons into the liposomes in response to the net electronegative charge across the liposome membranes (Deamer, D.W., et al., Biochim. et Biophys. Acta 274:323 (1972)).

In another more preferred approach, the proton gradient used for drug loading is produced by creating an ammonium ion gradient across the liposome membrane, as described, for example, in U.S. Patent No. 5,192,549. The liposomes are prepared in an aqueous buffer containing an ammonium salt, typically 0.1 to 0.3 M ammonium salt, such as ammonium sulfate, at a suitable pH, e.g., 5.5 to 7.5. The gradient can also be produced by using sulfated polymers, such as dextran ammonium sulfate or heparin sulfate. After liposome formation and sizing, the external medium is exchanged for one lacking ammonium ions, e.g., the same buffer but one in which ammonium sulfate is replaced by NaCl or a sugar that gives a similar osmolarity inside and outside of the liposomes.

Fig. 6 illustrates the ionization events in loading the exemplary nitorixide tempamine (TMN) into a liposome 10 against an ammonium ion gradient. After liposome formation, the ammonium ions inside the liposomes are in equilibrium with ammonia and protons. Ammonia is able to penetrate the liposome bilayer and escape from the liposome interior. Escape of ammonia continuously shifts the equilibrium within the liposome toward the left, to production of protons.

The nitroxide is loaded into the liposomes by adding the antioxidant to a suspension of the ion gradient liposomes, and the suspension is treated under conditions effective to allow passage of the compound from the external medium into the liposomes. Incubation conditions suitable for drug loading are those which (i) allow diffusion of the compound, typically in an uncharged form, into the liposomes, and (ii) preferably lead to high drug loading concentration, e.g., 2-500 mM drug encapsulated, more preferably between 2-200 mM.

The loading is preferably carried out at a temperature above the phase transition temperature of the liposome lipids. Thus, for liposomes formed predominantly of saturated phospholipids, the loading temperature may be as high as 60°C or more. The loading period is typically between 1-120 minutes, depending on permeability of the drug to the liposome bilayer membrane, temperature, and the relative concentrations of liposome lipid and drug.

With proper selection of liposome concentration, external concentration of added compound, and the ion gradient, essentially all of the compound may be loaded into the liposomes. For example, with a pH gradient of 3 units (or the potential of such a gradient employing an ammonium ion gradient), the final internal:external concentration of drug will be about 1000:1. Knowing the calculated internal liposome volume, and the maximum concentration of loaded drug, one can then select an amount of drug in the external medium which leads to substantially complete loading into the liposomes.

Alternatively, if drug loading is not effective to substantially deplete the external medium of free drug, the liposome suspension may be treated, following drug loading, to remove non-encapsulated drug. Free drug can be removed, for example, by molecular sieve chromatography, dialysis, or centrifugation.

In studies performed in support of the present invention, six liposome formulations were prepared and loaded with tempamine. Table 1 summarizes the lipid composition, liposome size and type, and the drug to lipid ratio for each formulation. Preparation of the liposomes is described in Example 2.

<div align="center">Table 1</div>

| No | Liposome composition[1] | Liposome type[2] and size (nm) | % encapsulation (remote loading) | tempamine/phos pho-lipid (mole ratio) |
|---|---|---|---|---|
| I | EPC FOR REFERENCE | MLV, 1200±200 | 85 | 0.09 |
| II | HPC FOR RE REFERENCE | MLV, 1200±200 | 85 | 0.09 |
| III | EPC:Chol:[2000]PEG-DSPE (54:41:5 mole ratio) | MLV, 1200±200 | 86 | 0.10 |
| IV | EPC:Chol:[2000]PEG-DSPE (54:41:5 mole ratio) | LUV, 100±20 | 96 | 0.12 |
| V | HPC:Chol:[2000]PEG-DSPE (54:41:5 mole ratio) | MLV, 1200±200 | 86 | 0.10 |
| VI | HPC:Chol:[2000]PEG-DSPE (54:41:5 le ratio) | LUV, 100±20 | 96 | 0.12 |

[1]EPC, egg phosphatidylcholine, Tm = -5°C; HPC, hydrogenated soy phosphatidylcholine, Tm = +52°C; Chol, cholesterol, [2000]PEG-DSPE, N-carbamyl-poly-(ethylene glycol methyl ether)-1,2-distearoyl-sn-glycero-3-phosphoethanolamine triethyl ammonium salt, average molecular mass of the PEG moiety was 2000 Da.
[2]MLV= multilamellar vesicles; LUV = large unilamellar vesicles

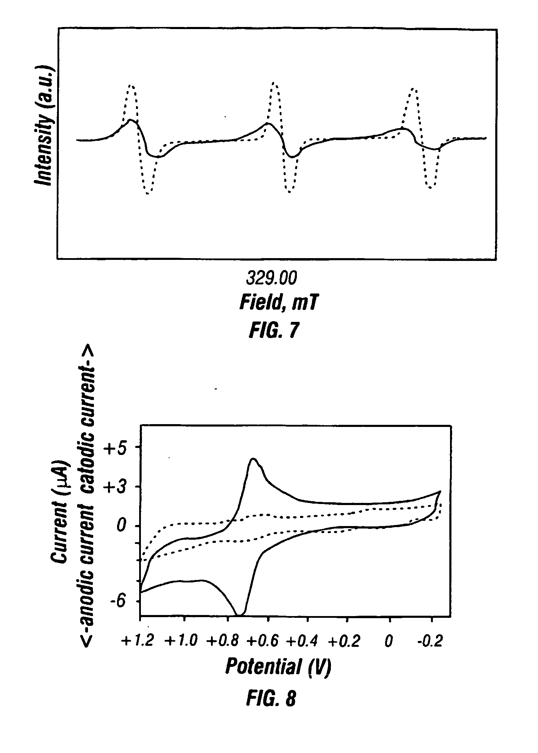

The kinetics of the tempamine remote loading process was examined during some of the loading processes using EPR and CV. EPR and CV measurements were performed during the remote loading process at intervals of 5, 10, 30, and 60 min. Fig. 7 shows the electron paramagnetic resonance (EPR) signal of tempamine before (dashed line) and after (solid line) encapsulation into liposomes. Fig. 8 shows the cyclic voltammetry (CV) signal of tempamine before (dashed line) and after (solid line) encapsulation into liposomes As can be seen from these figures, five minutes after loading, the tempamine signal changed dramatically. The spectra remained constant and no further changes were observed at the longer time points (data not shown). These results suggest that the tempamine loading process into the liposomes is fast, with the loading near completion by about 5 minutes.

IV. Liposome *In vitro* Characterization

A. Partition Coefficients

The n-octanol/ water partition coefficient, Kp, of tempamine was measured to estimate its phase distribution in the liposomes, according to the procedure previously described (Samuni, A. et al., Free Radic Biol Med., 22:1165 (1997)). Partition coefficients were measured at various pH levels (pH 4.0, 7.0, and 10.6), at tempamine concentrations of 2.0 mM and 20.0 mM and at different concentrations of ammonium sulfate (20-400 mM). The volume of each phase was 1 ml. The results are shown in Table 2.

**Table 2**

**Distribution of tempamine between *n*-octanol/aqueous phase at different pH's**

| Ammonium sulfate (mM) | TMN, ($\mu$mole ) | *n*-octanol/aqueous phase ($K$p), pH 10.6 | *n*-octanol/aqueous phase ($K$p), pH 7 | *n*-octanol/aqueous phase ($K$p), pH 4 |
|---|---|---|---|---|
| 0 | 20 | 2.331 | 0.278 | 0.111 |
| 20 | 2 | 2.418 | 0.048 | 0.031 |
| 20 | 20 | 2.386 | 0.116 | 0.074 |
| 150 | 2 | 2.184 | 0.055 | 0.024 |
| 150 | 20 | 3.969 | 0.038 | 0.049 |
| 400 | 2 | 3.005 | 0.040 | 0.028 |
| 400 | 20 | 4.569 | 0.034 | 0.046 |

At acidic and neutral pHs, high ammonium sulfate concentrations shift $K$p to the aqueous phase. At alkali pH, elevation in ammonium sulfate concentration shifts $K$p to *n*-octanol. This implies that at acidic and neutral pHs tempamine forms a complex with the sulfate ion, since otherwise the ammonium sulfate ions would shift the amphipathic molecules into the less polar phase (Standal, S.H. et al., J. Colloid Interface Sci., 212: 33 (1999)). These data also show that at lower pH values tempamine becomes concentrated in the aqueous phase. This is consistent with the fact that tempamine is a weak base and at acidic and neutral pHs tempamine is positively charged.

Piperedine nitroxides which have a similar chemical structure (TEMPO, TEMPOL) but do not have the charged amino group have much higher affinity to *n*-octanol than to the aqueous phase (Samuni, A., et al., Free Radic Biol Med., 22:1165 (1997)).

In another study, the partition of tempamine between the lipid bilayer and the aqueous phase was determined. The lipid bilayer/aqueous phase distribution was performed using a dialysis membrane separating the phases in 0.15 M NaCl (Samuni, A., et al., Free Radic Biol Med., 22:1165 (1997)). There was no predisposition of tempamine for the lipid bilayer over the 0.15 M NaCl aqueous phase and the distribution of tempamine was equal between the liposome preparation (10% egg phosphatidylcholine (EPC) (w/v)) and 0.15 M NaCl (data not shown). This indicates that there is no appreciable adsorption of tempamine to the neutral (EPC) liposome membrane and there is no significant tempamine penetration to the liposomes unless there is an ammonium sulfate gradient.

B. Percent Encapsulation

The amount of tempamine encapsulated in the liposomes was determined using EPR, as described in Example 3. As can be seen from Fig. 7, the EPR profile of encapsulated tempamine (solid line) is much broader and of lower intensity than that of an identical amount of free tempamine (dashed line) in aqueous solution. The data are summarized in Table 3.

**Table 3**

**Percent encapsulation of tempamine (TMN) in liposome preparations with and without ammonium sulfate gradient**

| Experiment No. | TMN (mM) | Liposomes | $NH_4^+$ gradient | Broadening agent | Nigericin | EPR signal $\pm$ S.D. (a. u.) | % Encapsulation $\pm$ S.D. |
|---|---|---|---|---|---|---|---|
| 1 | 0.2 | -[1] | - | - | - | 8.8$\pm$0.9 | |
| 2 | 0.2 | +[2] | + | - | - | 3.8$\pm$0.4 | |
| 3 | 0.2 | + | - | - | - | 8.2$\pm$0.8 | |
| 4 | 0.2 | + | + | + | - | 2.4$\pm$0.2 | 84.1$\pm$8.4[3] |
| 5 | 0.2 | + | - | + | - | 0.3$\pm$0 | 6$\pm$0.6 |

(continued)

| Percent encapsulation of tempamine (TMN) in liposome preparations with and without ammonium sulfate gradient | | | | | | | |
|---|---|---|---|---|---|---|---|
| Experiment No. | TMN (mM) | Liposomes | $NH_4^+$ gradient | Broadening agent | Nigericin | EPR signal ± S.D. (a. u.) | % Encapsulation ± S.D. |
| 6 | 0.2 | + | + | - | + | 8.7±0.9 | |

[1]"-" indicates the item in the top row was not included
[2]"+" indicates the item in the top row was included
[3]Example of calculations:
1. tempamine free=3.8 - 2.4 = 1.4;
2. TMN liposomes(nonquenched) = 8.7 - 1.4 = 7.3;
3. Percent encapsulation = 100x7.3/8.7x100 = 84.1; Quenching factor = 7.4/2.4 = 3.

These studies show that there is no tempamine loss during the remote loading procedure. Nigericin releases all the tempamine from the liposomes, as indicated by the fact that the tempamine signal after addition of nigericin and the signal of free tempamine of the same concentration are identical. Using equation 4 (see Example 3), the quenching factor was calculated to be approximately 3. The studies also show that the tempamine is active, as evidenced by the EPR functional assay.

C. Kinetics of Tempamine Release from Liposomes

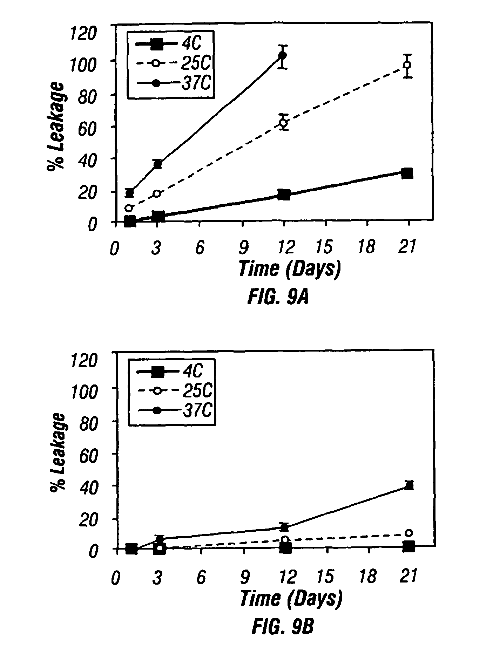

The release of tempamine from a suspension of liposomes comprised of either egg phosphatidylcholine (EPC) or hydrogenated soybean phosphatidylcholine (HPC) over a 21 day period was monitored, as described in Example 4. The release of tempamine into the aqueous external medium was determined at three temperatures, 4°C, 25°C, and 37°C. The results for liposomes comprised of EPC are shown in Fig. 9A, and for liposomes comprised of HPC in Fig. 9B.

As seen in Fig. 9A, at 4°C (squares), after 10 days, less than 20% of the entrapped tempamine had leaked from the liposomes, compared with 50% loss at 25°C (open circles) and more than 70% at 37°C (closed circles). After 3 weeks at 4°C, more than 70% remained encapsulated, while at 25°C, less than 10%. At 37°C, no encapsulated tempamine remained encapsulated after 3 weeks. The energy of activation (Ea) of tempamine release was 71 KJ/mole.

As seen in Fig. 9B, the release of tempamine from HPC liposomes was considerably slower than from the EPC liposomes. Less than 10% leakage was observed for HPC liposomes at 4°C (squares), 25°C (open circles), and 37°C (closed circles) after 10 days. After three weeks, no leakage was observed at 4°C; at 25°C less than 10% and at 37°C less than 50% leaked out. The energy of activation of tempamine release (Ea) was 43 KJ/mole.

Full recovery of the EPR signal after release of tempamine from liposomes proves that tempamine is released in the form of a fully functional stable radical. Both the CV data (above) the and EPR results show that tempamine does not lose its antioxidant activity after release from the liposomes.

As is clear from the data shown in Figs. 9A-9B, the release rate of the entrapped tempamine was affected by the $T_m$ of matrix lipid. In general, the release rate was lower for HPC than for EPC, and for multilamellar vesicles (MLV) than for large unilamellar (LUV) vesicles. For the other liposome formulations shown in Table 1, after 2 months, percent encapsulation was in the following order, where the roman numbers represent the formulation number in Table 1: V > VI > III > IV, where the percent encapsulation was 85% > 75% > 72% > 53%, respectively for these formulations.

D. Stability of Liposomes

The stability of liposomes in saline and human plasma was determined by diluting the suspension of liposomes from 10 mM initial concentration to 1 mM tempamine either with human plasma or with 0.15 M NaCl. The diluted liposomes were incubated at 37°C for 15 hours. The percent encapsulated tempamine was determined as described in Example 3. A control liposome dispersion was diluted with 0.15 M NaCl to 1 mM tempamine and immediately measured (time 0).

The results are summarized in Fig. 10 which shows the percent encapsulation and stability of four tempamine-loaded liposomal formulations as a function of lipid composition (refer to Table 1 for abbreviations) and liposome size. The percent encapsulation of tempamine immediately after liposome preparation (dotted bars), after 2 months storage in saline at 4°C (hatched bars), after 15 hours storage in saline (horizontal stripes), and after 15 hours in plasma at 37°C (white bars) is shown. In general, the leakage from MLV was not altered by plasma, compared to 0.15 M NaCl, as seen by comparing the HPC:Chol:[2000]PEG-DSPE liposome formulation and EPC:Chol:[2000]PEG-DSPE liposome formulation.

The difference between HPC-based and EPC-based liposome stability was much higher when large unilamellar vesicles (LUV) were compared. There was a difference in tempamine leakage from LUV when the extraliposomal medium was plasma or saline for both kinds of formulations (EPC-based and HPC-based). At 37°C, leakage in plasma was higher than in saline, as seen for EPC-based liposomes where a 92% leakage in plasma was observed, compared to 56% leakage in saline. For HPC-based liposomes a 29% leakage in plasma was observed, compared to 15% leakage in saline. Surprisingly, however, pharmacokinetic studies in mouse plasma demonstrated that tempamine entrapped in liposomes having hydrophilic polymer chains and a rigid lipid matrix, such as HPC, had an enhanced (prolonged) circulation lifetime. These studies, described in the following section, show that the half-life of tempamine in plasma was extended from several minutes to about six hours.

V. Liposome *In vivo* Characterization

To date, it has not been shown that tempamine also possesses antineoplastic activity. It is also unknown if tempamine can be successfully loaded and retained in a liposome *in vivo.* Remote loading and retention is desirable because remote loading achieves a high amount of drug in the intraliposomal aqueous phase almost independent of trapped volume. A high drug load would enable use of small unilamellar liposomes (SUV) which are capable of extravasating and accumulating in tumors, In this section, studies conducted in support of the invention showing that tempamine has antineoplastic activity and can be loaded and retained in SUVs to enhance blood circulation lifetime of tempamine for effective *in vivo* tumor treatment are described.

A. Pharmacokinetics and Biodistribution of Liposome-Entrapped Tempamine

The pharmacokinetics and biodistribution of liposome-entrapped tempamine and of free tempamine were determined in healthy and in tumor-bearing mice. As detailed in Example 5, normal BALB/c mice and BALB/c mice bearing subcutaneous implants of C26 tumor cells ($10^6$ cells/mouse) received 18 mg (105 µmole)/kg of liposome-entrapped tempamine or free tempamine by intravenous injection. The liposome formulation included a lipid label to allow analysis of the distribution of the liposome lipids. The tempamine levels in blood and tissues were measured using the EPR technique set forth in the Methods section.

The pharmacokinetic parameters of free tempamine and of liposome-entrapped tempamine after administration to mice are shown in Table 4. There was no apparent difference in tempamine elimination time and tissue distribution between normal and tumor-bearing mice; therefore only the results of tumor-bearing mice are presented.

**Table 4**

**Pharmacokinetic Parameters[1] of Liposome-entrapped Tempamine and Free Tempamine in Mice**

|  | $T_{1/2}$ (h) | CL (ml/h) | AUC (mg*h/ml) | Vss (ml) |
|---|---|---|---|---|
| liposome-entrapped | 7.90±0.85 | 0.15±0.005 | 2.63±0.29 | 1.52±0.07 |
| free drug Change fold | 0.15±0.009 52.7-↑ | 3.2±0.07 21.3↓ | 0.17±0.005 15.4↑ | 55±15.7 36.2↓ |

[1]calculated using WinNonlin analytical software, non-compartment analysis.
$T_{1/2}$=blood circulation half-life; CL=clearance rate; AUC=area under the curve; Vss=volume of distribution.

Fig. 11 is a plot showing the plasma elimination (percentage of injected dose) as a function of time after intravenous administration of 18 mg (105 µmole)/kg of tempamine in free form (closed circles) or in liposome-entrapped form (open circles). The elimination of free tempamine was fast compared to liposome-entrapped tempamine, as seen by comparing the half-life ($T_{1/2}$) in Table 4 and by comparing the elimination profiles shown in Fig. 11. A reduction in volume of distribution (Vss) was achieved by loading of tempamine into liposomes having a coating of polymer chains. The Vss of liposome-entrapped tempamine was 1.52 ml, slightly larger than the actual volume of mouse plasma. This indicates that liposome-entrapped tempamine remained in the plasma compartment after the injection and was not removed to peripheral compartments.

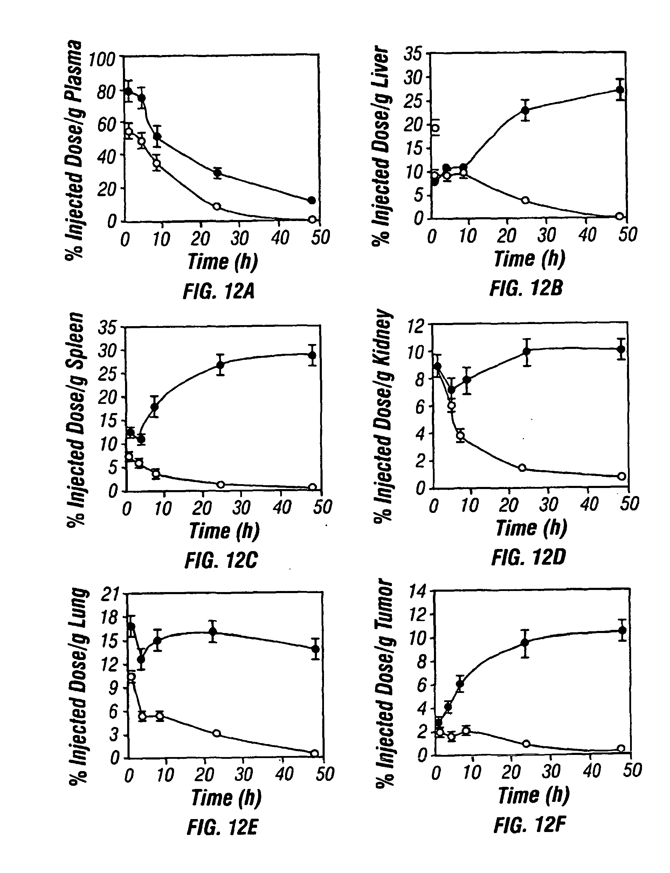

The results of the biodistribution analysis are shown in Figs. 12A-12F where the amount (percentage of injected dose) of tempamine (open circles) and of the liposomal lipid label (closed circles) plasma (Fig. 12A), liver (Fig. 12B), spleen (Fig. 12C), kidney (Fig. 12D), lung (Fig. 12E), and tumor (Fig. 12F) are shown. In this study, the mice were injected intravenously with 2 µmole/mouse liposome-entrapped tempamine and 14 umol/mouse phospholipid.

Fig. 12A shows that the liposome lipid and the liposome-entrapped tempamine were eliminated from plasma in similar pattern. A drop of radioactivity in the first 8 hours was observed, with a subsequent slowing of elimination rate until the final time point of 48 hours.

Figs. 12B-12F show the tissue distribution of liposome-entrapped tempamine and of the lipid label. In general, traces of free tempamine were observed in the liver and spleen at 1 hour and 4 hours after injection. In other organs, the levels of tempamine at the 4 hour time point was below the detection minimum (0.1 $\mu$M). Thus, the tissue distribution results presented in Figs 12B-12F are for liposome-entrapped tempamine only. The tempamine level in the liver (Fig. 12B, open circles) was stable between 1 to 8 hours after the injection. At 24 hours the tempamine level dropped to 25% of the initial level (at 1 hour) and after 48 hours 7.5% of the initial amount was detectable. The initially stable tempamine concentration in liver for the first 8 hours after injection may be attributed to a steady accumulation of liposome-entrapped tempamine in the liver. The lipid label [3H] Cholesteryl hexadecyl ether (closed circles) concentrations continuously increased over the 48 hour test period.

Fig. 12C shows the distribution of liposome-entrapped tempamine (open circles) and liposome label (closed circles) in the spleen. The tempamine level decreases over time, similar to the profile of tempamine elimination from plasma. This indicates there was no delayed tempamine accumulation in spleen. The lipid concentration (closed circles) as a function of time resembled that described for liver.

The tempamine level in the kidneys, as shown in Fig. 12D (open circles), decreased over time, indicating that no tempamine accumulation occurred in kidney. The lipid concentration (closed circles) in the kidneys was relatively constant at all tested time points indicating that liposomes accumulate in this organ, but to a lesser extent than in liver and spleen.

The highest concentration of tempamine was found in the lungs, as shown in Fig. 12E (open circles). One hour after injection 200 nmole/g tissue was measured. The level dropped to 50% of this value by four hours after administration with a slow decrease over the remaining test period. The drop in lung tempamine concentration was slower during the first 24 hours after injection than the tempamine level drop in plasma, suggesting that there was some tempamine accumulation in lungs during this time interval.

Fig. 12F shows the tempamine concentration (open circles) and the lipid concentration in the tumor tissue. The level of tempamine remained stable in tumor tissue between 1 to 8 hours after injection (42 nmole/g tissue). At 24 hours after administration the concentration decreased to about 18 nmole/g tissue. By the 48 hour time point the level was 4 nmole/g tissue. Tempamine clearance in tumor was slower than at all other tested tissues with 10% of the initial level (amount at 1-8 hour) still present 48 hours after injection. With respect to the labelled-lipid (closed circles), a continuous accumulation of radioactivity was observed over the test period, demonstrating that the liposome extravasate and accumulate into tumor tissue.

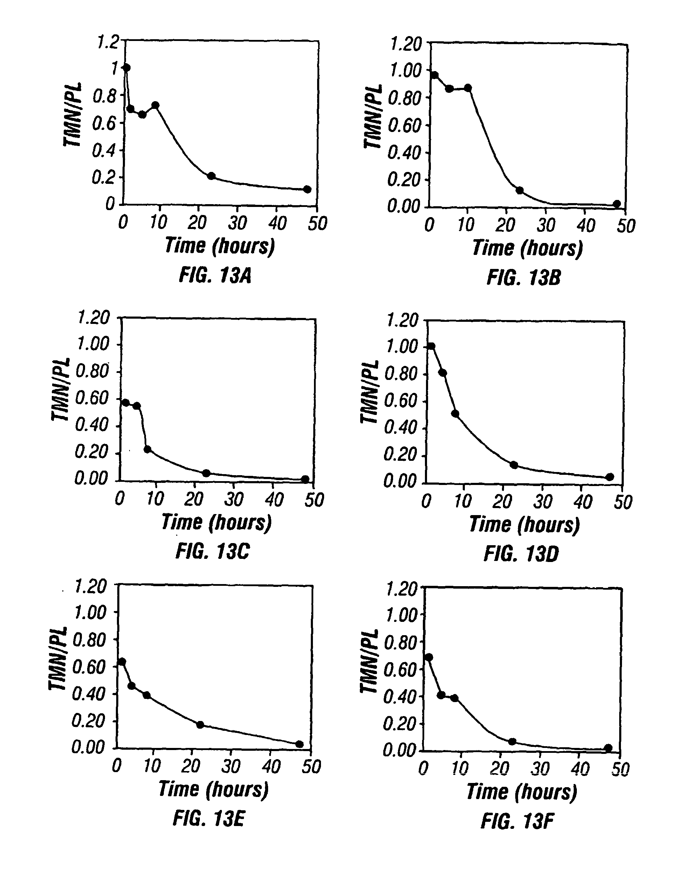

The leakage/release of drug from liposomes can be derived from the change in the mole ratio of drug to liposome (Amselem, S., et al., Chem. Phys. Lipids, 64:219 (1993)). This techniques was used to quantify release of tempamine from the liposomes in plasma and the results are shown in Figs. 13A-13F. The figures show the tempamine to phospholipid ratio in plasma (Fig. 13A), liver (Fig. 13B), spleen (Fig. 13C), kidney (Fig. 13D), lung (Fig. 13E), and tumor (Fig. 13F) at various times post injection.

In the plasma (Fig. 13A) within one hour after injection about 30% of the loaded tempamine had leaked out of the liposome into the plasma. After this initial burst, there was no significant drug leakage between the 1 hour to 8 hour time period after injection, suggesting the tempamine and [3H] cholesteryl ether elimination rates were the same. After 8 hours, a slow leakage of tempamine was observed. Despite the initial sharp drop in tempamine liposome payload, the stable and high amount of tempamine in liposomes for at least 8 hours provides a constant supply of intact liposome-entrapped tempamine to organs during this early post injection phase.

Figs. 13B-13F shows the tempamine to lipid ratio in various organs. As seen in Fig. 13B, the leakage rate in the liver was slow during first 8 hours after the injection and faster during the 8 to 24 hour period. In the spleen (Fig. 13C), the leakage was slow during first 4 hours after the injection and then accelerated. In the kidneys (Fig. 13D) the leakage rate was relatively constant over the test period. In the lungs (Fig. 13E) the leakage was relatively slow, compared to other organs. In the tumor tissue (Fig. 13F) the leakage was fast during first four hours after injection and was slowed (relative to other organs) thereafter.

VI. Utility of the Tempamine Composition

As discussed above, reactive oxygen species (ROS) can cause irreversible damage to cells and tissues. Many types of cancer cells have an altered oxidant level (Wiseman, H. et al., Biochem. J. 313:17-29 (1996)) and several tumors that have been strongly associated with the oxidant-antioxidant imbalance, including bladder, blood, bowel, breast, colorectal, liver, lung, kidney, esophagus, ovary, prostate, and skin. The generation of large amounts of reactive oxygen intermediates in cancer cells may contribute to the ability of some tumors to mutate, inhibit antiproteases, and injure local tissues, thereby promoting tumor heterogeneity, invasion, and metastasis. Accordingly, the invention contemplates the use of tempamine alone or in combination with other chemotherapeutic agents for the treatment of conditions characterized by cell proliferation.

Inflammation, both chronic and acute, is another pathology associated with damage resulting from ROS. Conditions arising from acute inflammation include UV-caused skin damage, non-steroidal antiinflammatory-drug-caused ulceritive colitis, and microbial or corrosive lung injury. Examples of pathologies where a chronic inflammation process is involved are presented in Table 5.

**Table 5**

| Pathological situation | Organ/System |
|---|---|
| alcoholism | liver |
| rheumatoid arthritis | joints |
| Behcet's disease | systemic, multi-orqan |
| Crohn's disease | digestive system |
| malaria | erythrocytes |
| adult respiratory distress syndrome | lung |

Arthritis, which takes place mostly in joints, is an example of a chronic inflammation process. In other studies performed in support of the present invention, the ability to target liposome-entrapped tempamine to inflamed tissues was evaluated using the adjuvant arthritis (AA) model in rats. AA is a T-cell-mediated autoimmune disease that can be induced in susceptible strains of rats, such as the Lewis strain (Ulmansky and Naparstek, Eur J. Immunol. 25(4):952-957, 1995). AA in rats is commonly used as an experimental model of rheumatoid arthritis and ankylosing spondylitis and for the testing of antiinflammatory and/or immunosuppressive drugs (Pearson, C.M., in McCarty D.J., Ed. ARTHRITIS AND ALLIED CONDITIONS, 9th Ed., Lea & Febiger, Philadelphia, p. 308, 1979).

Studies using the AA model are described in Example 6. In this study, AA was induced in male rats by injection of microbacteria in Freund's ajuvavnt. Liposomes containing tempamine were prepared as described in Example 5A by remote loading tempamine against an ammonium sulfate gradient. The liposomes were administered by injection to healthy and arthritic rats 22 days after inducement of AA. At regular time intervals after administration of the liposomes, plasma and tissue samples were taken to determine the biodistribution and pharmacokinetics. The results are summarized in Table 6A-6B.

**Table 6A**

| Recovery of Liposome-entrapped tempamine (based on EPR measurement) and liposomes (based on radioactivity measurements) in healthy rats. | | | | | | |
|---|---|---|---|---|---|---|
| **Organs** | 4 hours | | | 24 hours | | |
| | Liposome-entrapped TMN (% injected dose) | Liposome (% injected dose) | Ratio TMN/ Lipo [% release]] | Liposo me-entrapped TMN (% injected dose) | Liposome (% injected dose) | Ratio TMN/ Lipo [% release] |
| **Plasma** | **23.2** | **79** | **0.29 [71]** | **0** | **54** | **- [100]** |
| Liver | 1.5 | 2.4 | 0.62 [38] | 0 | 10 | **-[100]** |
| Lung | 0.32 | 0.43 | 0.74 [26] | 0 | 1.7 | **- [98]** |
| Spleen | 0.51 | 0.7 | 0.73 [27] | 0.12 | 5 | 0.024 **[100]** |
| Kidney | 0 | 1 | -[100] | 0 | 2.5 | **- [100]** |
| **Total** | **25.5** | **83.5** | **0.3 [70]** | **0.12** | **73.2** | **0.002 [100]** |

**Table 6B**

| Organs | 4 hours | | | 24 hours | | |
|---|---|---|---|---|---|---|
| | Liposome-entrapped TMN (% injected dose) | Liposome (% injected dose) | Ratio TMN/ Lipo [% release]] | Liposo me-entrapped TMN (% injecte d dose) | Liposome (% injected dose) | Ratio TMN/ Lipo [% release]] |
| Plasma | **30.40** | **81.00** | **0.37 [63]** | **4.31** | **58.00** | **0.074 [99]** |
| Liver | 1 | 2.35 | 0.43 [37] | 0 | 9.50 | - [100] |
| Lung | 0.42 | 0.67 | 0.62 [38] | 0 | 0.80 | - [100] |
| Spleen | 0.62 | 0.75 | 0.83 [17] | 0.18 | 2.80 | 0.064 [99] |
| Kidney | 0.67 | 0.7 | 0.96 [4] | 0 | 1.25 | - [100] |
| **Total** | **33.11** | **85.54** | **0.39 [61]** | **4.48** | **72.35** | **0.062 [99]** |

Table title: Recovery of Liposome-entrapped tempamine (based on EPR measurement) and liposomes (based on radioactivity measurements) in arthritic rats.

Four hours after injection of free tempamine, the amount of tempamine in plasma and in the tested tissues was below the detection limit (< 0.5 $\mu$M) in healthy and arthritic rats (data not shown). In contrast, the same dose of tempamine when injected in liposome-entrapped form results in about 41 $\mu$M (23% of the injected dose) in healthy and 53 $\mu$M (30% of injected dose) in arthritic rats present in the blood 4 hours after administration. At 24 hours post-injection, 4% of the injected dose was present in the plasma of arthritic rats (Table 6B). Traces of liposome-entrapped tempamine were detected in liver, spleen, and kidney at 4 hours and 24 hours after injection.

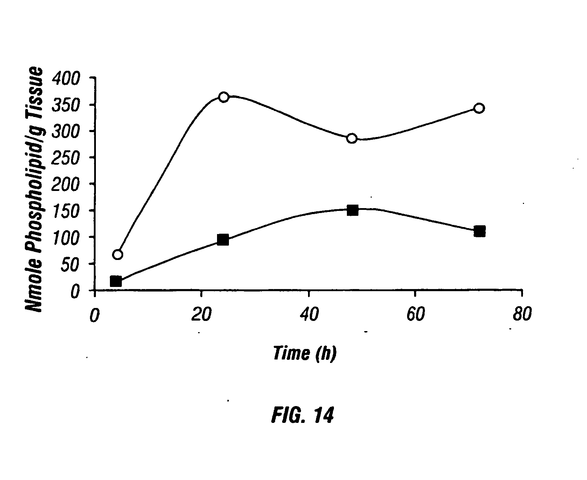

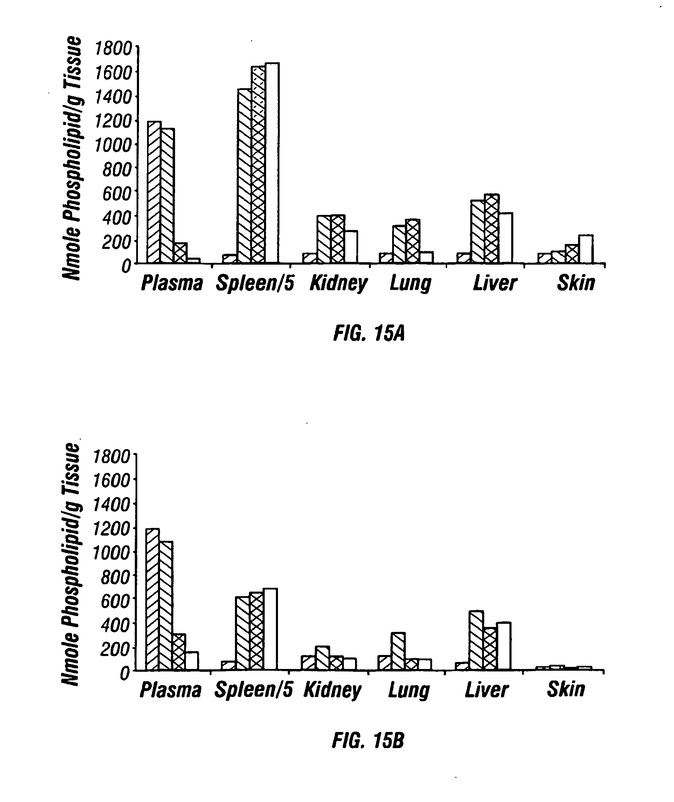

The ability of liposomes containing tempamine to extravasate selectively into inflamed tissues in healthy and arthritic rats were compared. The comparison is presented in Fig. 14. The tissue distribution of the liposomes and the plasma clearance rate in the healthy and arthritic rats was also determined, and the results are shown in Figs. 15A-15B.

Fig. 14 shows the amount (nmoles) of liposome phospholipid (measured using a radioactive lipid marker) per gram tissue, in healthy rats (closed circles) and in rats having induced adjuvent arthritis (open circles). A two-fold to four-fold higher extravasation of liposomes into the inflamed paws of arthritic rats relative to paws of healthy rats was observed at all time-points. The liposome concentration in the inflamed paws remained roughly unchanged from 24 hours to about 72 hours (= 220 $\mu$g lipid/g tissue; 293 $\mu$mole lipid/g tissue; 7% injected dose/paw). The liposome concentration in the paws of healthy rats was maximal at 48 hours (100 $\mu$g/g tissue or 2 % injected dose/paw).

Figs. 15A-15B are bar graphs showing the tissue distribution, taken as nmole phospholipid (PL)/gram tissue, of liposome-entrapped tempamine in healthy rats (Fig. 15A) and in rats having induced adjuvant arthritis (Fig. 15B) at 4 hours (dotted bars), 24 hours (hatched bars), 48 hours (horizontal stripes) and 72 hours (white bars) post-tempamine administration. Together with elevated liposome concentrations in the inflamed paw, liposome concentrations in skin, kidney, lung, and spleen of arthritic rats were lower than liposome concentrations in those tissues of healthy rats, suggesting that liposomes in arthritic rats were passively targeted and accumulated at the inflammation site.

The clearance rate of liposome-entrapped tempamine in both healthy and arthritic rats was significantly longer than free tempamine, with a half-life ($t_{1/2}$) of 23 hours in healthy rats and a half-life of 25 hours in arthritic rats, as was calculated using WinNolin analytical software.

A. <u>Combination Therapy</u>

In yet another aspect, the invention contemplates administration of tempamine in combination with chemotherapeutic agent. In studies performed in support of the invention, the ability of tempamine to act synergistically with other chemotherapeutic agents was demonstrated. Doxorubicin was chosen as a model chemotherpeutic agent. The enhancement of doxorubicin cytotoxicity by tempamine was tested on three cell lines, as described in Example 1A. A cytotoxicity assay as described in Example 1 B was used. Two of the cell lines, MCF-7 and M-109S, were doxorubicin-sensitive lines and one cell line, M-109R, was doxorubicin resistant. MCF-7 cells are more sensitive to tempamine (100 $\mu$M caused 75% growth inhibition), but are less sensitive to doxorubicin, than are M-109S cells. The results are summarized in Table 7.

Table 7

| Effect of tempamine concentrations on the $IC_{50}$ of doxorubicin (nM) on various cell lines | | | |
|---|---|---|---|
| **Cell Line** | **$IC_{50}$ of Doxorubicin (nM) in the presence of** | | |
| | **0 $\mu$M tempamine** | **50 $\mu$M tempamine** | **100 $\mu$M tempamine** | **200 $\mu$M tempamine** |
| **MCF-7** | 487$\pm$32 | 475$\pm$38 | 67$\pm$5.8 | 55$\pm$4.1 |
| **M-109S** | 60$\pm$4.0 | - | 27$\pm$1.7 | 18$\pm$1.1 |

As seen, in MCF-7 cells, the $IC_{50}$ value of doxorubicin decreased by one order of magnitude in the presence of 100 $\mu$M TMN. In M-109S cells, addition of 100-200 $\mu$M tempamine decreased to 50% the observed $IC_{50}$ of doxorubicin. In M-109R cells, addition of 200 $\mu$M tempamine enhanced cell sensitivity to doxorubicin.

In summary, relatively low tempamine concentrations were needed to increase cell sensitivity to doxorubicin. Combined treatment of cells with tempamine and doxorubicin significantly decreased the $IC_{50}$ of doxorubicin. This was particularly observed when cells were exposed to a low, non-cytotoxic concentration (100 $\mu$M) of tempamine.

From the foregoing, it can be seen how various objects and features of the invention are met. Tempamine, a piperidine nitroxide, has therapeutic activity as an agent effective to inhibit cellular growth and proliferation. The compound, administered alone or in a vehicle suitable to extend its blood circulation time, such as a liposome, is able to infiltrate into a diseased site, such as a tumor or an area of inflammation. In particular, delivery of the drug entrapped in a liposome, where the drug is loaded at high drug/lipid ratio in liposomes small enough for extravasation, provides a composition for treatment of conditions caused by oxidative damage. Tempamine is also effective to enhance the activity of other therapeutic agents, such as doxorubicin.

VII. <u>Examples</u>

The following examples further illustrate the invention described herein and are in no way intended to limit the scope of the invention.

## <u>Materials</u>

2,2,6,6-tetramethylpiperidine-4-amino-1-oxyl (4-amino-tempo, termed tempamine) free radical, 97%, was purchased from Aldrich (Milwaukee, WI, USA). Egg phosphatidylcholine (EPC I) and hydrogenated soybean phosphatidylcholine (HPC) were obtained from Lipoid KG (Ludwigshafen, Germany). *N*-carbamyl-poly-(ethylene glycol methyl ether)-1,2-distearoyl-*sn*-glycero-3-phosphoethanolamine triethyl ammonium salt ($^{2000}$PEG-DSPE) (the polyethylene moiety having a molecular mass of 2000 Da) was prepared conventionally. Cholesterol was obtained from Sigma (St. Louis, MO, USA). Sephadex G-50 was obtained from Pharmacia (Uppsala, Sweden). *tert*-Butanol was purchased from BDH, Poole, UK. Fluoroscein phosphatidylethanolamine was obtained from Avanti Polar Lipids (Alabaster, AL, USA). Other chemicals, including buffers, were obtained from Sigma. Dialysis membrane (dialysis tubing-visking (size 6-27/32") was obtained from Medicell International (London, UK). Purified water (WaterPro PS HPLC/Ultrafilter Hybrid model, Labconco, Kansas City, MO, USA) which provides lowest possible levels of total organic carbon and inorganic ions was used in all water-based preparations.

## <u>Methods</u>

### 1. <u>Electron paramagnetic resonance (EPR) measurements</u>

EPR spectrometry was employed to detect tempamine concentration using a JES-RE3X EPR spectrometer (JEOL Co., Japan) (Fuchs, J., *et al.*, *Free Radic. Biol. Med.,* 22:967-976, (1997)). Samples were drawn by a syringe into a gas-permeable Teflon capillary tube of 0.81 mm i.d. and 0.05 mm wall thickness (Zeus Industrial Products, Raritan, NJ, USA). The capillary tube was inserted into a 2.5-mm-i.d. quartz tube open at both ends, and placed in the EPR cavity. EPR spectra were recorded with center field set at 329 mT, 100 kHz modulation frequency, 0.1 mT modulation amplitude, and nonsaturating microwave power. Just before EPR measurements, loaded liposomes were diluted with 0.15 M NaCl for the suitable tempamine concentration range (0.02-0.1 mM). The experiment was carried out under air, at room temperature. This is a functional assay which determines the activity of tempamine.

## 2. Cyclic voltammetry (CV) measurements

All cyclic voltammograms were performed between - 200 mV and 1.3 V.

Measurements were carried out in phosphate-buffered saline, pH 7.4. A three-electrode system was used throughout the study. The working electrode was a glassy carbon disk (BAS MF-2012, Bioanalytical Systems, W. Lafayette, IN, USA), 3.3 mm in diameter. The auxiliary electrode was a platinum wire, and the reference electrode was Ag/AgCl (BAS). The working electrode was polished before each measurement using a polishing kit (BAS PK-1) (Kohen, R., et al., Arch. Gerontol. Geriat., 24:103-123, (1996)). Just before CV measurements the samples were diluted with buffer to the optimal tempamine concentration range (0.05-0.2 mM). The experiments were carried out under air, at room temperature. The CV assay is a functional assay.

## EXAMPLE 1

### *In vitro* Testing of Free Tempamine

A. Cell lines and Culture Conditions

MCF-7 (human breast adenocarcinoma), M-109S (doxorubicin-sensitive human breast carcinoma), and M-109R (doxorubicin-resistant human breast carcinoma) were maintained in RPMI medium (Biological Industries, Beit HaEmek, Israel) supplemented with 10% fetal calf serum (FCS). The cell lines were maintained under standard culture conditions at 37°C in a humidified 5% $CO_2$ atmosphere.

B. Cytotoxicity Assay

The effect of tempamine on cell proliferation was determined by the methylene blue assay (Horowitz, A.T., et al., Biochim Biophys Acta. 1109:203, (1992)). Briefly, cells were seeded onto 96-well plates (MCF cells at a density of $6 \times 10^3$ cells per well, and M-109S and M-109R at a density of $1.5 \times 10^3$ cells per well) and allowed to grow for 24 hours prior to treatment with different concentrations of tempamine. After the addition of tempamine ($5 \times 10^{-5}$ to $4 \times 10^{-4}$), the cells were incubated in RPMI + 10% FCS for four days without change of medium. Then the cells were fixed with 2.5% glutaraldehyde, stained with methylene blue and assayed spectrophotometrically.

C. Apoptosis detection

Apoptosis was assessed by flow cytometry (FACScan). $1 \times 10^6$ cells were removed from culture, washed with PBS, and stained with merocyanine-540 (Reid, S., et al., J. Immunol. Methods 192:43 (1996). Briefly, the cell pellet was resuspended in 500 µl PBS. 2.5 µl of a 1 mg/ml solution of merocyanine-540 was added to the cells, incubated for 2 min at room temperature in the dark. The cells were washed, resuspended in 1 ml PBS, and run immediately on a fluorescence-activated cell-sorting flow cytometer (Vantage, Becton Dickinson, Rutherford, NJ, USA).

## EXAMPLE 2

### Liposome Preparation

A. Liposome Formation

Liposomes were prepared by dissolving the lipid(s) (see Table 1 for the lipids used in each of the six formulations) in *tert*-butanol and lyophilized overnight. The dry lipid powder was resuspended with ammonium sulfate solution (150 mM). Rehydration was carried out above the $T_m$ of the matrix lipid: for HPC. 52.2°C and for EPC, -5°C (Marsh, D., Chem. Phys. Lipids, 57:109-120 (1991)). Rehydration was performed under continuous shaking, forming multilamellar vesicles (MLV). The volume of hydration medium was adjusted to obtain a 10% (w/v) lipid concentration. Large unilamellar vesicles (LUV) were prepared by extrusion of MLV through 0.1 µm-pore-size filters (Poretics, Livermore, CA, USA) using the LiposoFast-Basic device (AVESTIN, Ottawa, ON, Canada). The distribution of liposome sizes in the preparation was measured by photon correlation spectroscopy using a Coulter (Model N4 SD) submicron particle analyzer. Size distributions of $1200 \pm 200$ nm and $100 \pm 10$ nm were obtained for MLV and LUV, respectively. The liposome formulations used in the study are summarized in Table 1.

B. Formation of ammonium sulfate radient

The dialysis procedure of Amselem et al. (J. Liposome Res., 2:93-123 (1992)) was utilized. In brief, the procedure used two consecutive dialysis exchanges against 100 volumes of 0.15 M NaCl (pH = 5.2), and a third dialysis exchange against 100 volumes of 0.15 M KCl (pH = 6.7). Ammonium sulfate was dissolved at concentrations sufficient to give the desired gradients of $[(NH_4)_2SO_4]$ inside the liposomes over that in the external medium in the range of 100-1000.

C. Liposome loading with Tempamine

A concentrated tempamine alcoholic solution (0.8 ml of 25 mM tempamine in 70% ethanol) was added to 10 ml of liposomal suspension. The final solution contained 5.6% ethanol and 2 mM tempamine. Loading was performed above the $T_m$ of the matrix lipid. Loading was terminated at the specified time by removal of unencapsulated tempamine using the dialysis at 4°C. Loading efficiency was determined as described below.

## EXAMPLE 3

### Percent Encapsulation of Tempamine

The amount of entrapped tempamine of liposomes prepared according to Example 2 was determined by the following procedure. First, the total tempamine in the post-loading liposome preparation ($TMN_{mix}$) was measured. Then, the amount of tempamine in the post-loading liposome preparation in the presence of potassium ferricyanide, an EPR broadening agent that eliminates the signal of free (non-liposomal) tempamine, was measured. The remaining signal is of tempamine in liposomes ($TMN_{liposome(quenched)}$). This spectrum was broad, as tempamine concentration inside the liposomes was high, leading to quenching of its EPR signal due to spin interaction between the tempamine molecules which are close to one another. Then the total tempamine after releasing it from liposomes by nigericin ($TMN_{nigencin}$) was measured. This signal was identical to the total tempamine used for loading ($TMN_{nigerin}=TMN_{total}$) and is completely dequenched. $TMN_{liposome\ (not\ quenched)}$ represents the signal of liposomal tempamine when the ammonium sulfate gradient is collapsed and all the tempamine is released.

The percent encapsulation and the quenching factor were calculated as follows:

$$TMN_{free} = TMN_{mix} - TMN_{liposomes(quenched)} \qquad (1)$$

$$TMN_{liposomes(not\ quenched)} = TMN_{nigericin} - TMN_{free} \qquad (2)$$

$$Percent\ encapsulation = 100 \times TMN_{liposome(not\ quenched)}/TMN_{nigericin} \qquad (3)$$

$$Quenching\ factor = TMN_{liposome(not\ quenched)}/TMN_{liposome(quenched)} \qquad (4)$$

The data are summarized in Table 3.

## EXAMPLE 4

### Tempamine Release from Liposomes

The release of tempamine from egg phosphatidylcholine (EPC)-based liposomes and from hydrogenated soy phosphatidylcholine (HPC)-based liposomes prepared as described above was followed for 21 days at three different temperatures: 4°C, 25°C and 37°C. The pH of the liposomal dispersion medium was -5.5. From the liposomal suspension an aliquot was taken at defined times and the non-encapsulated tempamine was removed by gel filtration using a Sephadex-G50 column. The liposomes were placed in test tubes and stored at the specified temperature.

Before the EPR measurements all the samples were brought to room temperature (23°C). Each sample was measured first without and then with potassium ferricyanide, the EPR broadening agent, which eliminates the external tempamine signal arising from tempamine that has leaked from the liposomes following the gel filtration step. Percent encapsulation was calculated using equations 1-3 set forth in Example 3. The results are summarized in Figs. 9A-9B.

## EXAMPLE 5

### *In vitro* Testing of Liposome-Entrapped Tempamine

A. Liposome Preparation

Sterically stabilized liposomes composed of HPC:Chol:2000PEG-DSPE; 54:41:5 mole ratio, and a trace amount of [3H] cholesteryl ether (100 $\mu$Ci per 800 $\mu$mol phospholipid) were prepared as described by Gabizon et al.(Cancer Res., 54:987 (1994)). Briefly, the lipid components were dissolved in tert-butanol and then [3H] cholesteryl ether was added. A "dry cake" was formed by lyophilization overnight. The hydration medium consisted of 0.25 M ammonium sulfate (pH 5.7). Hydration was performed under vigorous vortexing at 60°C (above $T_m$ of the matrix lipid). Liposomes were downsized by extrusion at 60°C through double-stacked polycarbonate membranes of gradually decreasing pore size (0.4, 0.2, 0.1, 0.08, 0.05 $\mu$m) using a highpressure extrusion device (Lipex Biomembranes, Vancouver, BC, Canada). Extruded liposomes were dialyzed against a 100-fold volume of 0.15 M NaCl (four changes over 24 h) at 4°C.

Tempamine was loaded actively into the liposomes by an ammonium sulfate gradient. Loading was performed at 60°C, *i.e.* above $T_m$ of the matrix lipid, and stopped at the desired time by decreasing the temperature. The liposomal tempamine preparation was sterilized by filtration through a 0.2-$\mu$m-pore filter and stored at 4°C.

Phospholipid concentration was determined using a modification of Bartlett's procedure (Barenholz, Y., et al., in LIPOSOME TECHNOLOGY, G. Gregoriadis (Ed.), 2nd Edn., Vol. I, CRC Press, Boca Raton, pp. 527-616, (1993)). [3H] cholesteryl hexadecyl ether was measured by $\beta$-counting (KONTRON Liquid Scintillation Counter). Tempamine concentration in tissues and plasma was measured by electron paramagnetic resonance (EPR) as described above in the methods section. The distribution of liposome size in the preparation was measured by photon correlation spectroscopy using a Coulter (Model N4 SD, submicron particle analyzer). The phosopholipid loss after liposome preparation was 28%, with most of it occurring during extrusion. The amount of loaded tempamine was calculated using the EPR method described above. The loaded tempamine:phospholipid molar ratio obtained was approximately 0.14. Mean vesicle size was $88 \pm 15$ nm.

B. Biodistribution Studies

8 to12-week-old BALB/c female mice, obtained through the Animal Breeding House of the Hebrew University (Jerusalem, Israel), were used throughout the study. Animals were housed at Hadassah Medical Center with food and water ad libitum. All procedures were in accordance with the standards required by the Institutional Animal Care and Use Committee of the Hebrew University and Hadassah Medical Organization. Each mouse was injected with one inoculum of tumor cells ($1 \times 10^6$ C26 cells) subcutaneously into the left flank. One week after inoculation, tempamine 0.36 mg (2.1 $\mu$mole)/mouse (18 mg (105 $\mu$mole)/kg body weight) in free form or liposome-entrapped tempamine, was injected by intravenous (i.v.) bolus through the tail vein. Phospholipid dose was 11 mg (14.7 $\mu$mole)/mouse (377 mg (514 $\mu$mole)/kg body weight). At 1, 4, 8, 24, and 48 hours after the injection, the animals were anesthetized with ether inhalation, bled by eye enucleation, and immediately sacrificed for removal of liver, lung, spleen, kidney, and tumor. Each time point consisted of 3 mice. Plasma was separated by centrifugation.

C. Sample Preparation

To measure the total tempamine concentration (encapsulated and free) in the organs, the liposomes were solubilized by homogenization in a Polytron homogenizer (Kinematica, Lutzern, Switzerland) in 2% Triton X-100 (1:2, organ:Triton X-100 solution). The homogenized mixture was cooled and heated several times to destroy the lipid membrane (Barenholz, Y., et al., in LIPOSOME TECHNOLOGY, G. Gregoriadis (Ed.), 2nd Edn., Vol. I, CRC Press, Boca Raton, pp. 527-616, (1993)). The plasma samples were mixed 1:1 with 2% Triton X-1 00 to give the 1% Triton X-1 00 in the tested sample and also cooled and heated several times. These conditions were effective to achieve a release of all entrapped tempamine from intact liposomes.

For determination of the total concentration of nitroxide + hydroxylamine, potassium ferricyanide at a final concentration of 2-3 mM (depending on the tested tissue) was added to all the samples (plasma and organ homogenates) to oxidize the hydroxylamine to intact nitroxide.

D. [3H] Cholesteryl hexadecyl ether measurements in plasma and organs

From the samples prepared as described above in section F, duplicates of 100 $\mu$l were burned in a Sample Oxidizer (Model 307, Packard Instrument Co., Meridien, CT) and left overnight in a dark, cool place. The samples were then measured by $\beta$-counting (KONTRON Liquid Scintillation Counter).

## EXAMPLE 6

**Liposome-Entrapped Tempamine for Treating Arthritis**

A. <u>Animals</u>

Male Lewis rats (160-180 g) were purchased from Harlan Sprague-Dawley, Indianapolis, IN. They were housed in a controlled environment and provided with standard rodent chow and water.

Adjuvent arthritis (AA) was induced by a single intradermal injection of mycobacteria in mineral oil (Freund's adjuvant). In strains of rats susceptible to adjuvant arthritis, the non-specific primary inflammation at the injection site was followed on about the 10th post-injection day by a disseminated polyarthritis or secondary specific inflammation. Lewis strain rats, which are highly susceptible to AA, were injected with 1 mg of Mycobacterium tuberculosis H37Ra (Difco, Detroit, MI, USA) in Freund's complete adjuvant (FCA) (Difco), subcutaneously at the base of the tail. Maximum swelling of the paw occurred between 20 and 27 days

B. <u>Liposomes</u>

Liposomes were prepared as described in Example 5A.

C. <u>Biodistribution and Pharmacokinetics</u>

Free tempamine or liposome-entrapped tempamine were injected into healthy and arthritic rats 22 days after injection of Freund's Complete Adjuvent (maximum swelling). Tempamine was administered at a dose of 1.8 mg tempamine/kg (10.5 $\mu$mol/kg). The phospholipid concentration of the injected liposomes was 42 mg/kg (56 $\mu$mol/kg). At 4 hours, 24 hours, 48 hours and 72 hours after injection some rats in each test groups were sacrificed and their plasma, liver, kidneys, spleen, and lungs were tested for liposomal marker [3H] cholesteryl hexadeyl ether (in whole tissue) using a Sample Oxidiser (Model 307, Packard Instrument Co., Meriden, CT), and for tempamine (in tissue homogenate with addition of 2% Triton to solubilize the liposome) using a JES-RE3X EPR spectrometer (JEOL Co., Japan). Skin and paws were tested for presence of the liposomal marker. The results are presented in Tables 6A-6B.

Although the invention has been described with respect to particular embodiments, it will be apparent to those skilled in the art that various changes and modifications can be made without departing from the invention.

## Claims

1. A composition comprising liposomes comprised of

   (i) a vesicle-forming lipid;
   (ii) between about 1 to 20 mole percent of a lipid derivatized with a hydrophilic polymer, and
   (iii) tempamine entrapped in said liposomes.

2. The composition according to claim 1, wherein said tempamine is entrapped in the liposome at a concentration of between 2 and 500 mM.

3. The composition according to claim 1, wherein the tempamine is precipitated in the presence of sulphate as counterion.

4. The composition according to any one of claims 1 to 3, wherein the liposome further comprises cholesterol.

5. The composition according to any one of claims 1 to 4, wherein said vesicle-forming lipid is hydrogenated phosphatidylcholine.

6. The composition according to any one of claims 1 to 5, wherein said hydrophilic polymer is polyethylene glycol.

7. A composition according to any one of claims 1 to 6 for use in treating cancer or acute and chronic inflammation, comprising liposomes according to any one of claims 1 to 6.

**Patentansprüche**

1. Zusammensetzung, die Liposomen umfasst, die umfassen

   (i) ein Vesikel-bildendes Lipid;
   (ii) zwischen etwa 1 bis 20 Molprozent eines Lipids, das mit einem hydrophilen Polymer derivatisiert ist, und
   (iii) Tempamin, das in den Liposomen gefangen ist.

2. Zusammensetzung gemäß Anspruch 1, wobei das Tempamin bei einer Konzentration zwischen 2 und 500 mM in den Liposomen gefangen ist.

3. Zusammensetzung gemäß Anspruch 1, wobei das Tempamin in Gegenwart von Sulfat als Gegenion präzipitiert ist.

4. Zusammensetzung gemäß einem der Ansprüche 1 bis 3, wobei das Liposom ferner Cholesterin umfasst.

5. Zusammensetzung gemäß einem der Ansprüche 1 bis 4, wobei das Vesikel-bildende Lipid hydriertes Phosphatidylcholin ist.

6. Zusammensetzung gemäß einem der Ansprüche 1 bis 5, wobei das hydrophile Polymer Polyethylenglykol ist.

7. Zusammensetzung gemäß einem der Ansprüche 1 bis 6, die Liposomen gemäß einem der Ansprüche 1 bis 6 umfasst, zur Verwendung in der Behandlung von Krebs oder akuter und chronischer Entzündung.


**Revendications**

1. Composition comprenant
   des liposomes composés de

   (i) un lipide formant des vésicules ;
   (ii) entre environ 1 et 20 moles pour cent d'un lipide dérivatisé avec un polymère hydrophile, et
   (iii) de la tempamine piégée dans lesdits liposomes.

2. Composition selon la revendication 1, dans laquelle ladite tempamine est piégée dans le liposome à une concentration comprise entre 2 et 500 mM.

3. Composition selon la revendication 1, dans laquelle la tempamine est précipitée en présence de sulfate comme contre-ion.

4. Composition selon l'une quelconque des revendications 1 à 3, dans laquelle le liposome comprend en outre du cholestérol.

5. Composition selon l'une quelconque des revendications 1 à 4, dans laquelle ledit lipide formant des vésicules est une phosphatidylcholine hydrogénée.

6. Composition selon l'une quelconque des revendications 1 à 5, dans laquelle ledit polymère hydrophile est un polyéthylène glycol.

7. Composition selon l'une quelconque des revendications 1 à 6, pour son utilisation dans le traitement du cancer ou d'une inflammation chronique et aiguë, comprenant des liposomes selon l'une quelconque des revendications 1 à 6.

**FIG. 1A**

**FIG. 1B**

**FIG. 1C**

**FIG. 2**

329.00

Field, mT

FIG. 3

Concentration, molar X 10$^{-4}$

FIG. 4

FIG. 5A

FIG. 5B

$(NH_4)_2SO_4$

$2NH^3 \rightleftarrows 2NH_3 + 2H^+ \rightleftarrows 2NH_4 + SO_4^{2-}$ $2TMN-NH_3^+ + 2Cl^-$

$(TMN-NH_3)_2SO_4 \rightleftarrows 2TMN-NH_2 \rightleftarrows 2TMN-NH_2 + 2H^+$

FIG. 6

329.00
Field, mT
FIG. 7

Potential (V)
FIG. 8

FIG. 9A

FIG. 9B

FIG. 10

FIG. 11

FIG. 12A

FIG. 12B

FIG. 12C

FIG. 12D

FIG. 12E

FIG. 12F

FIG. 13A

FIG. 13B

FIG. 13C

FIG. 13D

FIG. 13E

FIG. 13F

**FIG. 14**

FIG. 15A

FIG. 15B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4179337 A
- US 5380532 A, Chakrabarti, A.
- US 5013556 A
- WO 9807409 A
- US 5395619 A
- US 6043094 A
- US 5192549 A

### Non-patent literature cited in the description

- FREE RADICAL IN BIOLOGY AND MEDICINE. Clarendon Press, 1989
- PARKE et al. *Int. J. Occup. Med. Environ. Health,* 1996, vol. 9, 331-340
- KNIGHT. *Ann. Clin. Lab. Sci.,* 1997, vol. 27, 11-25
- STOHS. *J. Basic. Clin. Physiol. Pharmacol.,* 1995, vol. 6, 205-228
- SAMUNI, A. et al. *Free Radical Res. Commun.,* 1991, vol. 12-13, 187-194
- SAMUNI, A. et al. *J. Clin. Invest,* 1991, vol. 87, 1526-1530
- BURTON, G.W. et al. *J. Am. Chem. Soc.,* 1981, vol. 103, 6478-6485
- MITCHELL, J.B. et al. *Arch. Biochem. Biophys.,* 1991, vol. 298, 62-70
- MATES, J.M. et al. *Int. J. Biochem. Cell Biol.,* 2000, vol. 32, 157-170
- WISEMAN, H. et al. *Biochem. J.,* 1996, vol. 313, 17-29
- MATES, J.M. et al. *Int J Biochem. Cell Biol.,* 2000, vol. 32, 157-170
- SZATROWSKI, T.P. et al. *Cancer Res.,* 1991, vol. 51, 794-798
- SHACTER, E. et al. *Blood,* 2000, vol. 96, 307-313
- LEE, Y-J. et al. *J. Biol. Chem.,* 1999, vol. 274, 19792-19798
- SAVILL, J. et al. *Immunol. Today,* 1993, vol. 14, 131-136
- CHENERY, R. et al. *Nat. Med.,* 1997, vol. 3, 1233-1241
- NICHOLS, J.W. et al. *Biochim. Biophys. Acta,* 1976, vol. 455, 269-271
- CRAMER, J. et al. *Biochemical and Biophysical Research Communications,* 1977, vol. 75 (2), 295-301
- MADDEN, T.D. et al. *Chemistry and Physics of Lipids,* 1990, vol. 53, 37-46
- AFZAL, V. et al. *Invest. Raiol.,* 1984, vol. 19, 549-552
- ANKEL, E.G. et al. *Life Sci.,* 1987, vol. 40, 495-498
- DEGRAFF, W.G. et al. *Environ. Mol. Mutagen.,* 1992, vol. 19, 21-26
- MITCHELL, J.B. et al. *Arch. Biochem. Biophys,* 1991, vol. 298, 62-70
- SAMUNI, A. et al. *Free Radical. Res. Commun.,* 1991, vol. 12-13, 187-194
- MITCHELL, J.B. et al. *Tempol. Arch. Biochem. Biophys.,* 1991, vol. 298, 62-70
- KRISHNA, M.C. et al. *Proc Natl Acad Sci U S A.,* 1992, vol. 89, 5537-5541
- KOCHERGINSKY, N. ; SWARTZ, H.M. NITROXIDE SPIN LABELS: REACTIONS IN BIOLOGY AND CHEMISTRY. CRC Press, 1995
- REID, S. et al. *J. Immunol. Methods,* 1996, vol. 192, 43
- DEAMER, D.W. et al. *Biochim. et Biophys. Acta,* 1972, vol. 274, 323
- SAMUNI, A. et al. *Free Radic Biol Med.,* 1997, vol. 22, 1165
- STANDAL, S.H. et al. *J. Colloid Interface Sci.,* 1999, vol. 212, 33
- AMSELEM, S. et al. *Chem. Phys. Lipids,* 1993, vol. 64, 219
- ULMANSKY ; NAPARSTEK. *Eur J. Immunol.,* 1995, vol. 25 (4), 952-957
- ARTHRITIS AND ALLIED CONDITIONS. Lea & Febiger, 1979, 308
- KOHEN, R. et al. *Arch. Gerontol. Geriatr.,* 1996, vol. 24, 103-123
- HOROWITZ, A.T. et al. *Biochim Biophys Acta,* 1992, vol. 1109, 203
- MARSH, D. *Chem. Phys. Lipids,* 1991, vol. 57, 109-120
- AMSELEM et al. *J. Liposome Res.,* 1992, vol. 2, 93-123
- GABIZON et al. *Cancer Res.,* 1994, vol. 54, 987
- BARENHOLZ, Y. et al. *LIPOSOME TECHNOLOGY,* 1993, vol. I, 527-616

• **BARENHOLZ, Y. et al.** LIPOSOME TECHNOLOGY. CRC Press, 1993, vol. I, 527-616